# EUROPEAN PATENT APPLICATION

(11) **EP 1 452 601 A1**
(43) Date of publication of application: **01.09.2004**
(21) Application number: 03004326.9
(22) Date of filing: 28.02.2003
(51) Int. Cl.: C12N 15/62, C07K 1/107, C12N 15/63, C12N 15/10

(54) **Enhanced expression of fusion polypeptides with a biotinylation tag**

(71) Applicant: Roche Diagnostics GmbH, 68305 Mannheim (DE); F.HOFFMANN-LA ROCHE AG, 4070 Basel (CH)
(72) Inventor: The designation of the inventor has not yet been filed

(57) **Abstract**

The invention provides the means to enhance in E.coli-based expression systems the formation of fusion polypeptides containing as an N-terminal tag a biotinylation polypeptide. By way of specifically exchanging in the nucleic acid sequence encoding the biotinylation polypeptide nucleotides at 11 discrete positions enhances the formation of the total fusion polypeptide by at least 40%.

## Description

### Field of the invention

The present invention relates to nucleic acids encoding a polypeptide capable of being biotinylated by holocarboxylase synthetase. In particular, the present invention relates to the formation of fusion polypeptides comprising an N-terminal polypeptide capable of being biotinylated by holocarboxylase synthetase and a C-terminal polypeptide with a biological function. More particularly, the invention relates to the enhanced formation of such fusion polypeptides by means of expression in vitro or in vivo E.coli-based expression systems. The invention therefore relates to the field of molecular biology, but given the diverse uses for recombinant proteins, the invention also relates to the fields of chemistry, pharmacology, biotechnology, and medical diagnostics.

### Background of the invention

The enzyme holocarboxylase synthetase of E.coli (BirA, a biotin ligase) catalyzes in vivo the biotinylation, that is the covalent addition of biotin to the ε-amino group of a lysine side chain in its natural substrate, biotin carboxyl carrier protein (BCCP) (Cronan, J.E., Jr., et al., J. Biol. Chem. 265 (1990) 10327-10333). In E.coli only BCCP is biotinylated. This protein is a subunit of acetyl-CoA carboxylase. The reaction is catalysed by the biotin-protein ligase, the product of the BirA gene (Cronan, J.E., Jr., Cell 58 (1989) 427-429).

A BirA substrate consisting of a sequence of 13 amino acids was defined as a biotinylation polypeptide in fusion polypeptides (Schatz, P.J., Biotechnology 11 (1993) 1138-1143). WO 95/04069 describes biotinylation peptides that can be fused to other peptides or proteins of interest using recombinant DNA techniques. The resulting fusion polypeptides can be biotinylated in vivo or in vitro by BirA holocarboxylase synthetase. Particularly WO 95/04069 describes the expression of such fusion polypeptides in E.coli and anticipates expression in cell-free expression systems. But both documents are completely silent regarding the impact of the nucleic acid sequence that is encoding an N-terminal biotinylation polypeptide on the expressed quantity of the fusion polypeptide.

US 5,723,584, US 5,874,239, US 5,932,433 and US 6,265,552 provide further amino acid sequences for biotinylation polypeptides to be used for generating fusions with polypeptides of interest. Regarding N-terminally tagged fusion polypeptides, the documents describe the chemical synthesis of nucleic acid sequences that were biased in order to fit a consensus biotinylation polypeptide sequence. However, the documents are completely silent regarding the impact of the nucleic acid sequence that is encoding an N-terminal biotinylation polypeptide on the expressed quantity of the fusion polypeptide.

The biotinylation polypeptide used in the present invention (SEQ ID NO: 1, AviTag™) is comprised in the pAN-4, pAN-5, pAN-6 series of expression vectors distributed by Avidity Inc., Denver, Colorado, USA. The set of 3 different pAN vectors are designed for cloning and expression of N-terminal tagged fusion polypeptides in each reading frame. The DNA sequence encoding the biotinylation polypeptide is the DNA sequence of SEQ ID NO: 3.

Moreover, a synthetic BirA biotinylation polypeptide that was identified by combinatorial methods and consisted of a sequence of 23 amino acids was used to define a minimum sequence required for biotinylation that consisted of a sequence of 14 amino acids (Beckett, D., et al., Protein Sci. 8 (1999) 921-929). The 14-mer was proposed to mimic the acceptor function of BCCP as the natural BirA substrate. The impact of the nucleic acid sequence encoding biotinylation polypeptide on the expressed quantity of the fusion polypeptide was not investigated.

US 6,326,157 describes the construction of fusion polypeptides consisting of green fluorescent protein tagged with a biotinylation polypeptide. However, the document is completely silent regarding the impact of the nucleic acid sequence that is encoding an N-terminal biotinylation polypeptide on the expressed quantity of the fusion polypeptide.

E.coli-based cellular expression systems are well-known to the art and include US 5,232,840 regarding an optimised ribosome-binding site. Particularly cellular E.coli expression systems using the T7 promoter are described in US 4,952,496, US 5,693,489 and US 5,869,320.

Codon usage is one of the best known parameters impacting on the expressed quantity of a polypeptide. Genes in both prokaryotes and eukaryotes show a non-random usage of synonymous codons. The systematic analysis of codon usage patterns in E. coli led to the following observations (de Boer, H.A., and Kastelein, R.A., In: Maximizing gene expression, Reznikoff, W.S., and Gold, L., (eds.), Butterworths, Boston, 1986, pp. 225-285): (1) There is a bias for one or two codons for almost all degenerate codon families. (2) Certain codons are most frequently used by all different genes irrespective of the abundance of the protein. (3) Highly expressed genes exhibit a greater degree of codon bias than do poorly expressed ones. (4) The frequency of use of synonymous codons usually reflects the abundance of their cognate tRNAs. These observations imply that heterologous genes enriched with codons that are rarely used by E. coli may not be expressed efficiently in E. coli.

However, it appears to be difficult to generally and unambiguously predict whether the content of low-usage codons in a specific gene might adversely affect the efficiency of its expression in E.coli. Regarding the efficiency of translation of a polypeptide in E.coli, several influencing factors are superimposed, e.g. positional effects of certain codons, the clustering or interspersion of the rarely used codons, as well as the secondary structure of the mRNA. Nevertheless, from a practical point of view, the codon context of specific genes can have adverse effects on the quantity of expressed polypeptide levels. Usually, this problem is rectified by the alteration of the codons in question, whereby codons in the entire coding sequence are addressed. Another way to address this problem is to co-express the cognate tRNA genes (Makrides, S.C., Microbiol. Rev. 60 (1996) 512-538).

It is also known for in vitro translation systems that adding tRNAs that pair with rarely used codons can increase the expressed quantity of a polypeptide. An example for an in vitro translation system is the RTS 500 System that is distributed by Roche Diagnostics GmbH, Mannheim, Germany (catalogue number 3246817). In this expression system that comprises E.coli lysates, transcription and translation take place simultaneously in a reaction compartment of the reaction device. Substrates and energy components essential for a sustained reaction are continuously supplied via a semipermeable membrane. At the same time, potentially inhibitory reaction by-products are diluted via diffusion through the same membrane into the feeding compartment. Polypeptide is expressed for up to 24 hours yielding up to 5 mg of polypeptide.

Both, for cellular and for cell-free expression systems it is unclear if and to what extent the nucleic acid sequence encoding an N-terminal tag, such as a biotinylation polypeptide, alone can impact on the expressed quantity of a fusion polypeptide. Therefore, the problem to be solved is to provide the means to further enhance in a cell-free as well as in a cellular expression system the formation of a fusion polypeptide that comprises a biotinylation polypeptide.

### Brief summary of the invention

The invention provides the means to enhance in E.coli-based expression systems the formation of fusion polypeptides containing as an N-terminal tag a biotinylation polypeptide. It was surprisingly found that specifically exchanging in the nucleic acid sequence encoding the biotinylation polypeptide nucleotides at 11 discrete positions enhances the formation of the total fusion polypeptide by at least 40%.

Therefore, in a first aspect, the invention therefore provides nucleic acids encoding a polypeptide capable of being biotinylated by holocarboxylase synthetase. In a further aspect, the invention provides an expression vector comprising a nucleic acid according to the invention. In yet a further aspect, the invention provides a method of preparing a biotinylated polypeptide in a cell-free polypeptide synthesis reaction mixture. In yet a further aspect, the invention provides use of a nucleic acid according to the invention for constructing, by way of genetic engineering, a nucleic acid encoding a fusion polypeptide and expressing the same, whereby the fusion polypeptide consists of an N-terminal polypeptide capable of being biotinylated by holocarboxylase synthetase, and a C-terminal polypeptide with a biological function.

### Detailed description of the invention

Certain terms are used with particular meaning, or are defined for the first time, in this description of the present invention. For the purposes of the present invention, the following terms are defined by their art-accepted definitions, when such exist, except that when those definitions conflict or partially conflict with the definitions set forth below. In the event of a conflict in definition, the meaning of the terms are first defined by the definitions set forth below.

The term "comprising" is used in the description of the invention and in the claims to mean "including, but not necessarily limited to".

As used herein, the term "polypeptide with a biological function" refers to a polypeptide which possesses a biological function or activity which is identified through a defined functional assay and which is associated with a particular biologic, morphologic, or phenotypic alteration in a cell or a virus. Examples for polypeptides with a biological function are receptors, transcription factors, kinases, polypeptide subunits of complexes, or antibodies.

The term "polypeptide with a biological function" also encompasses "functional fragments" thereof, thus including all fragments of a the polypeptide with a biological function that retain an activity of the polypeptide. Functional fragments, for example, can vary in size from a polypeptide fragment as small as, e.g., an epitope capable of binding an antibody molecule to a large polypeptide capable of participating in the characteristic induction or programming of phenotypic changes within a cell.

Minor modifications of the primary amino acid sequences of a "polypeptide with a biological function" may result in polypeptides which have substantially equivalent activity as compared to the unmodified counterpart polypeptide. Such modifications may be deliberate, as by site-directed mutagenesis, or may be spontaneous. Further, C- or N-terminal addition of one or more amino acids, insertion of one or more amino acids, as well as deletion of one or more amino acids can also result in a modification of the structure of the resultant molecule without significantly altering its activity. All of the polypeptides produced by these modifications are included under the term "polypeptide with a biological function" as long as the biological activity of the polypeptide still exists.

Additionally, the term "polypeptide with a biological function" encompasses a hybrid polypeptide, that is to say a fusion of two or more polypeptides with biological functions.

The term "polypeptide" denotes a polymer composed of amino acid monomers joined by peptide bonds. A "peptide bond" is a covalent bond between two amino acids in which the α-amino group of one amino acid is bonded to the α-carboxyl group of the other amino acid. All amino acid or polypeptide sequences, unless otherwise designated, are written from the amino terminus (N-terminus) to the carboxy terminus (C-terminus). Amino acid identification uses the three-letter abbreviations as well as the single-letter alphabet of amino acids, i.e. Asp D Aspartic acid, Ile I Isoleucine, Thr. T Threonine, Leu L Leucine, Ser S Serine, Tyr Y Tyrosine, Glu E Glutamic acid, Phe F Phenylalanine, Pro P Proline, His H Histidine, Gly G Glycine, Lys K Lysine, Ala A Alanine, Arg R Arginine, Cys C Cysteine, Trp W Tryptophan, Val V Valine, Gln Q Glutamine, Met M Methionine, Asn N Asparagine.

The term "biotinylation polypeptide" is a "polypeptide capable of being biotinylated by holocarboxylase synthetase". The amino acid sequence of the biotinylation polypeptide provides a sequence motif containing an acceptor site for "biotinylation", that is the covalent attachment of a biotin molecule by holocarboxylase synthetase.

As used herein, the term "tagging" or "tagging a target sequence" refers to introducing by recombinant methods a nucleic acid encoding a "tag" such as a biotinylation polypeptide into a polypeptide-encoding nucleic acid, i.e. a "target sequence" so that the recombinant nucleic acid encodes a fusion polypeptide which comprises the tag at its C- or N-terminus.

The term "fusion polypeptide" refers to a polypeptide which has been tagged, e.g. with a biotinylation polypeptide. For example, the amino acid sequence of a fusion polypeptide may comprise the amino acid sequence of the biotinylation polypeptide and the amino acid sequence of a target polypeptide. The target polypeptide itself is a polypeptide with a biological function.

"Nucleic acid" as used herein refers to DNA or RNA which may be single- or doublestranded, and represents the sense strand when single-stranded. Nucleic acids are polymers with nucleotides as monomers. Nucleotides are composed of a phosphate moiety, a sugar moiety (ribose or deoxyribose) and an aglyconic heterocyclic moiety, the so-called nucleobase. In a nucleic acid sequence a single letter defines a nucleotide by its nucleobase, i.e. adenine (A), guanine (G), cytosine (C) and thymine (T) or uracil (U).

Nucleic acids encoding fusion polypeptides can be prepared by chemical methods or by genetic engineering. A fusion polypeptide can be obtained by means of "expression" of a nucleic acid encoding the same, that is as a result of transcription and translation the nucleic acid.

A nucleic acid is "operably linked" when it is placed into a functional relationship with another nucleic acid. For example, a nucleic acid encoding a biotinylation polypeptide is operably linked to a nucleic acid encoding a polypeptide with a biological function if it is expressed as a fusion polypeptide capable of being biotinylated; a promoter is operably linked to a coding sequence if it affects the transcription of the sequence; or a ribosome binding site is operably linked to a coding sequence if it is positioned so as to facilitate translation. Generally, operably linked means that the nucleic acids being linked are contiguous and, in the case of a nucleic acid encoding, e.g., a biotinylation polypeptide, contiguous and in reading phase. As for DNA, linking is accomplished by ligation at convenient restriction sites. If such sites do not exist then synthetic oligonucleotide adaptors or linkers are used in accord with conventional practice.

All nucleic acid sequences are written in the direction from the 5' (stands for prime) end to the 3' end also referred to as 5' to 3'. The nucleic acid sequences of the invention that encode a polypeptide of SEQ ID NO: 1 are different from previously published nucleic acid sequences such as SEQ ID NO: 3 because of the degeneracy of the genetic code and encode the same polypeptide. Degenerate code stands for a genetic code in which a particular amino acid can be coded by two or more different codons. Degeneracy occurs because of the fact that of the 64 possible base triplets, 3 are used to code the stop signals, and the other 61 are left to code for only 20 different amino acids.

The term "expression system" is well understood in the art to mean either an in vitro system or cellular or multicellular organism capable of translating or transcribing and translating nucleotide sequences to produce polypeptides. An example for an in vitro expression system, that is to say a cell-free polypeptide synthesis reaction mixture, is described in Zubay, G., Annu. Rev. Genet. 7 (1973) 267-287. Spirin et al. developed in 1988 a continuous-flow cell-free translation and coupled transcription/translation system in which a relatively high amount of protein synthesis occurs (Spirin, A.S., et al., Science 242 (1988) 1162-1164). Examples of application of such systems are documented by Pratt, J.M., et al., Nucleic Acids Research 9 (1981) 4459-4479, and Pratt et al., In: Transcription and Translation: A Practical Approach, Hames and Higgins (eds.), 1984, pp. 179-209, IRL Press. Further developments of the cell-free protein synthesis are described in US 5,478,730, US 5,571,690, EP 0932664, WO 99/50436, WO 00/58493, and WO 00/55353. Cellular expression systems that are based on E.coli are described in US 5,232,840, US 4,952,496, US 5,693,489 and US 5,869,320.

A first aspect, the invention provides a nucleic acid of SEQ ID NO: 2 encoding a polypeptide of SEQ ID NO: 1 capable of being biotinylated by holocarboxylase synthetase, characterised in that said nucleic acid differs from SEQ ID NO: 3 by nucleotide exchanges at 6 or more positions selected from the group consisting of the positions 4, 5, 6, 9, 10, 12, 15, 18, 21, 24 or 30, and said nucleic acid, as compared to SEQ ID NO: 3, enhances the formation of a fusion polypeptide, consisting of an N-terminal polypeptide according to SEQ ID NO: 1 and a C-terminal polypeptide with a biological function, by means of expression from a nucleic acid encoding said fusion polypeptide in a cell-free polypeptide synthesis reaction mixture in that at least 40% more fusion polypeptide is formed, whereby the nucleic acid encoding said fusion polypeptide consists of a nucleic acid encoding said N-terminal polypeptide operably linked to a nucleic acid encoding said C-terminal polypeptide.

In a preferred embodiment of the invention, the nucleic acid that is containing A or T at position 4, C or G at position 5, C or T at position 6, A, C or T at position 9, C or T at position 10, A or G at position 12, C or T at position 15, C or T at position 18, C or T at position 21, C or T at position 24, and A or T at position 30, is characterised in that between 5 and 11 nucleotides at said positions are identical to the nucleotides at the same positions in SEQ ID NO: 4, with the proviso that all nucleotides at said positions are identical to the nucleotides at the same positions in SEQ ID NO: 3 or SEQ ID NO: 4, or 10 nucleotides at said positions except position 9 are identical to the nucleotides at the same positions in SEQ ID NO: 4 or SEQ ID NO: 3, and the nucleotide at position 9 is T.

In another preferred embodiment of the invention, the nucleic acid is characterised in that the nucleic acid is selected from the group consisting of SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23 or SEQ ID NO: 24.

Another aspect of the invention is an expression vector comprising a nucleic acid according to the invention.

Yet another aspect of the invention is a method of preparing a biotinylated polypeptide in a cell-free polypeptide synthesis reaction mixture which contains an RNA polymerase, ribosomes, tRNA, ATP, GTP, nucleotides and amino acids, comprising the steps of (a) forming in said reaction mixture a fusion polypeptide, consisting of an N-terminal polypeptide according to SEQ ID NO: 1 and a C-terminal polypeptide with a biological function, by means of expression from a nucleic acid consisting of a nucleic acid according to any of the claims 1 to 3 operably linked to a nucleic acid encoding the C-terminal polypeptide; (b) biotinylating said fusion polypeptide in the presence of biotin and holocarboxylase synthetase; (c) isolating said biotinylated fusion polypeptide from said mixture; or incubating said mixture with immobilised avidin or streptavidin under such conditions that said biotinylated fusion polypeptide is bound to said immobilised avidin or streptavidin.

A preferred RNA polymerase is a DNA-dependent RNA polymerase. A very much preferred RNA polymerase is T7 RNA polymerase.

Holocarboxylase synthetase (EC6.3.4.15, biotin protein ligase, BirA) is an enzyme that catalyses in E.coli the covalent attachment of biotin to its natural substrate, that is BCCP. Biotin ligase is highly specific and reacts only on biotinylation polypeptides showing a very high degree of conservation in the primary structure of the biotin attachment domain. This domain includes preferably the highly conserved AMKM tetrapeptide (Chapman-Smith, A., and Cronan, J.E., Jr., J. Nutr. 129, 2S Suppl., (1999) 477S-484S). Recombinant BirA enzyme is described in WO 99/37785. In order to biotinylate fusion polypeptides, holocarboxylase synthetase can be added to an in vitro expression system as an active enzyme or can be added as a nucleic acid (in an expression vector, e.g. RNA, DNA) which is expressed (transcribed/translated) in the system like the fusion polypeptide.

Therefore, in a preferred embodiment of the invention, the method is characterised in that the reaction mixture contains a nucleic acid encoding holocarboxylase synthetase according to SEQ ID NO: 25 that is expressed in said reaction mixture to provide holocarboxylase synthetase polypeptide. If added as an active enzyme, it is used preferably in an amount of about 10,000 to 15,000 units, preferably 12,500 units. A preferred active enzyme (EC6.3.4.15) is supplied by Avidity Inc. (Denver, Colorado, USA).

In another preferred embodiment of the invention, the method is characterised in that the reaction mixture contains a nucleic acid encoding holocarboxylase synthetase according to SEQ ID NO: 25 that is expressed in the reaction mixture to provide holocarboxylase synthetase polypeptide. The amount of nucleic acid depends on the expression rate of the used vector and the necessary amount of BirA enzyme in the reaction mixture. 1 ng of BirA plasmid DNA (e.g. on the basis of a commercially available E.coli expression vector such as pIVEX vectors, supplied by Roche Diagnostics GmbH, Mannheim, Germany; http://www.biochem.roche.com/RTS), or even less, is sufficient for a quantitative biotinylation reaction of the tagged fusion polypeptides. The maximum yield of expressed and specifically biotinylated fusion polypeptide is achieved, when the desired fusion polypeptide-encoding plasmid DNA is added at 10-15µg and the plasmid DNA, being responsible for the coexpression of BirA, is introduced with an amount between 1-10ng. The ratio of fusion polypeptide-encoding plasmid DNA to BirA-encoding plasmid DNA was found to be optimal at a ratio of about 1500:1. It was found that the same level as above is sufficient for quantitative biotinylation of the expressed fusion protein. D(+)-biotin was added at 1 to 10 µM, preferably in about 2 µM to the reaction mixture.

After the expression of the fusion polypeptide in the cell-free expression system, biotinylation occurs under standard reaction conditions, preferably within 10 to 30 hours at 20°C to 36°C, most preferably at about 30°C, and the reaction mixture is preferably, after dialysis, for concentration and buffer exchange, centrifuged.

In a preferred embodiment of the invention, the solution is, due to its high purity, directly used for immobilisation of the fusion polypeptide on surfaces which contain immobilised avidin or streptavidin (e.g. microtiter plates or biosensors) without further purification.

According to the invention it is possible to produce highly pure biotinylated polypeptides which can be bound to surfaces in ligand binding experiments, e.g. surface plasmon resonance spectroscopy or ELISA assays.

If required, biotinylated polypeptides produced according to the present invention can be purified further under native conditions using matrices containing immobilised (preferably monomeric) avidin, streptavidin, or derivatives thereof. A variety of useful physically (Kohanski, R.A., and Lane, M.D., Methods Enzymol. 184 (1990) 194-200), chemically (Morag, E., et al., Anal. Biochem. 243 (1996) 257-263) and genetically (Sano, T., and Cantor, C.R., Proc. Natl. Acad. Sci.USA 92 (1995) 3180-3184) modified forms of avidin or streptavidin have been described that still bind biotin specifically but with weaker affinity to facilitate a one step purification procedure.

Yet another aspect of the invention is use of a nucleic acid according to any of the claims 1 to 3 for constructing, by way of genetic engineering, a nucleic acid encoding a fusion polypeptide, whereby the fusion polypeptide consists of an N-terminal polypeptide of SEQ ID NO: 1 and a C-terminal polypeptide with a biological function. Methods for constructing by way of genetic engineering are well known to the art and are described, in e.g. Sambrook, Fritsch & Maniatis, Molecular Cloning, A Laboratory Manual, 3rd edition, CSHL Press, 2001.

Yet another aspect of the invention is use of a nucleic acid according to the invention for expressing a fusion polypeptide, whereby the fusion polypeptide consists of an N-terminal polypeptide of SEQ ID NO: 1 and a C-terminal polypeptide with a biological function.

A preferred embodiment of the invention is the use characterised in that the fusion polypeptide is expressed in a cell-free polypeptide synthesis reaction mixture. A preferred cell-free polypeptide synthesis reaction mixture is the RTS500 in vitro expression system supplied by Roche Diagnostics (Mannheim, Germany; catalogue number 3246817).

Another preferred embodiment of the invention is the use characterised in that the fusion polypeptide is expressed in E.coli. A preferred E.coli strain is a BL21 (DE 3) strain. Even more preferred is a BL21 (DE 3) Lys S strain. These strains express an active T7 RNA polymerase. Such a strain can be used to transcribe a gene carried by an expression vector, whereby the vector comprises, e.g., a nucleic acid encoding a fusion polypeptide that is operably linked to the T7 promoter. Examples for vectors that have incorporated the T7 promoter and that are capable of being transcribed in the BL21 (DE 3) strain or the BL21 (DE 3) Lys S strain of E.coli are pET vectors (Novagen Inc., Madison, Wisconsin, USA) or pIVEX vectors (Roche Diagnostics GmbH, Mannheim, Germany). Methods for expressing fusion polypeptides are well known to the art and are described (e.g. in: Sambrook, Fritsch & Maniatis, Molecular Cloning, A Laboratory Manual, 3rd edition, CSHL Press, 2001. Also in: Gu, J., et al., Biotechniques 17 (1994) 257, 260, 262).

The following examples, references, sequence listing and figures are provided to aid the understanding of the present invention, the true scope of which is set forth in the appended claims. It is understood that modifications can be made in the procedures set forth without departing from the spirit of the invention.

### Description of the Figures

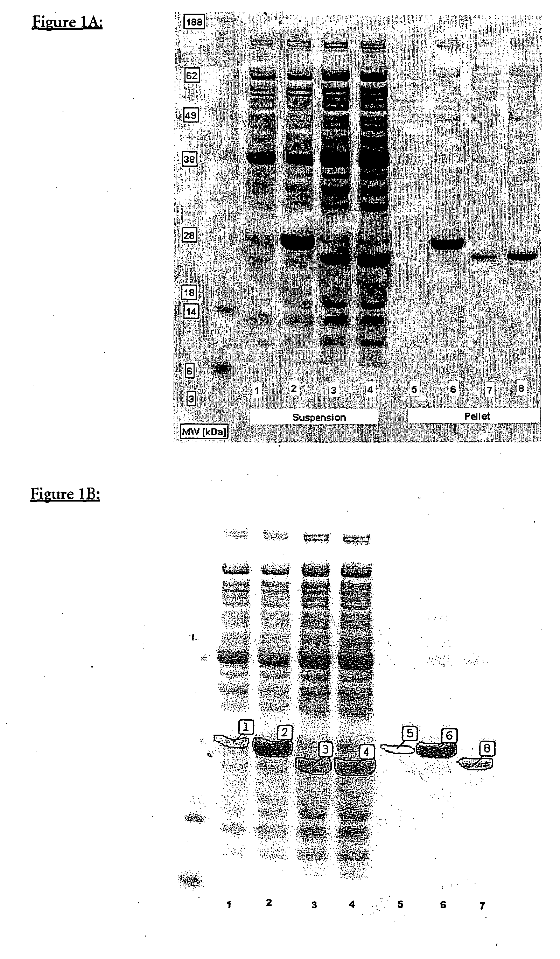

- **Figure 1A**: In vitro expression (see Example 3) of fusion polypeptides from pIVEX-2.8 CAT WT Avitag with the wildtype sequence encoding the N-terminal tag (lane 1, 5), pIVEX-2.8 CAT mut Avitag with the sequence of SEQ ID NO: 12 encoding the N-terminal tag (lane 2, 6), pIVEX-2.8 EPO WT Avitag with the wildtype sequence encoding the N-terminal tag (lane 3, 7), pIVEX-2.8 EPO mut Avitag with the sequence of SEQ ID NO: 12 encoding the N-terminal tag (lane 4, 8). The total protein suspension of each cell-free polypeptide synthesis reaction mixture was applied in lanes 1-4, the pellet fraction in lane 5-8.
- **Figure 1B**: Densitometric analysis as described in Example 4 was performed on the areas indicated.

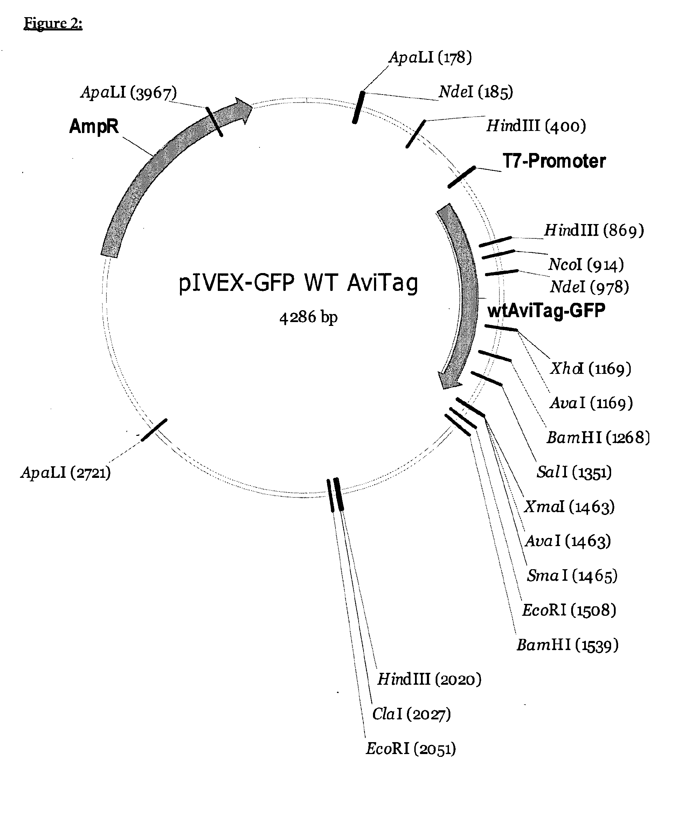

- **Figure 2**: pIVEX-GFP WT Avitag

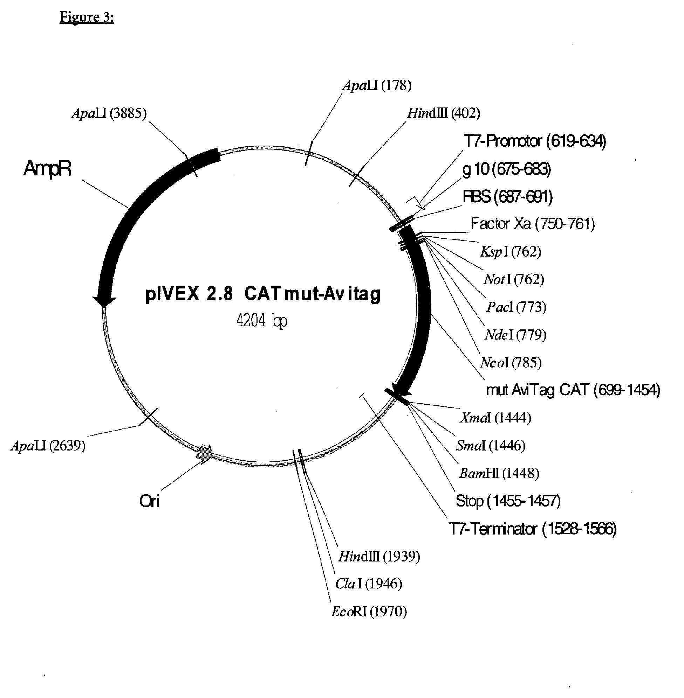

- **Figure 3**: pIVEX-2.8 CAT mut Avitag; the site denoted "Xa factor" indicates a cleavage site for factor Xa protease.

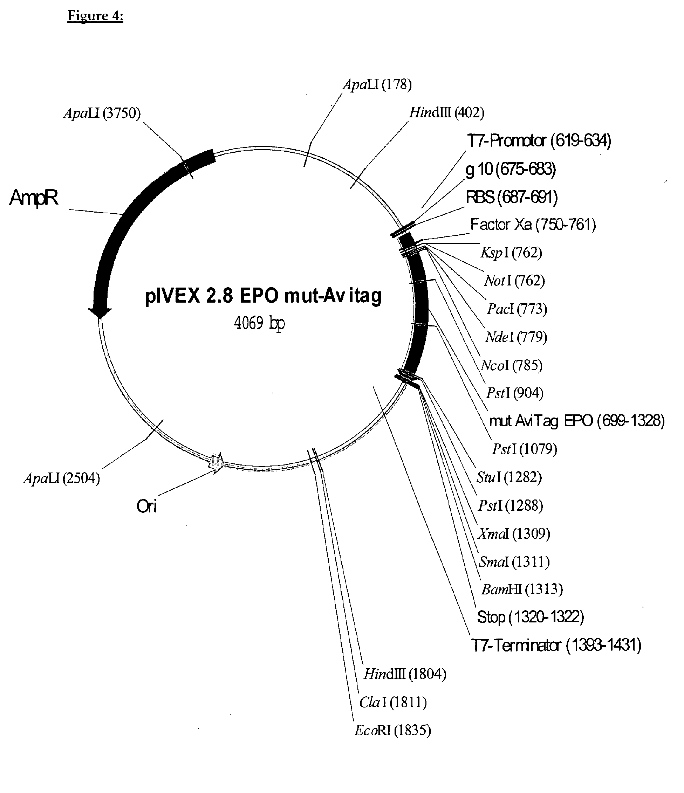

- **Figure 4**: pIVEX-2.8 EPO mut Avitag; the site denoted "Xa factor" indicates a cleavage site for factor Xa protease.

### Example 1

### Mutant variants of the DNA sequence encoding the AviTag™ biotinylation polypeptide

The AviTag™ biotinylation polypeptide comprises a sequence of 15-17 amino acid residues and can be used as a tag in fusion polypeptides. The AviTag™ is capable of being biotinylated at a lysine residue by a biotin protein ligase such as the polypeptide encoded by the E.coli BirA gene (Murtif, V.L., and Samols, D., J. Biol. Chem. 262 (1987) 11813-11816). The AviTag™ biotinylation polypeptide used for the present invention is represented by SEQ ID NO: 1. A DNA sequence encoding the AviTag™ and expression vectors in which the DNA sequence is incorporated are commercially available from Avidity Inc. (Denver, Colorado, USA). The original DNA sequence of which variants were generated is SEQ ID NO: 3. This sequence is also referred to as "wildtype sequence" or "wildtype DNA sequence".

For the purpose of generating optimised mutant variants of the AviTag™ encoding DNA sequence, that is to say variants that enhance the expression of a fusion polypeptide that comprises the AviTag™ biotinylation polypeptide, the wildtype DNA sequence was placed in-frame in front of the test protein green fluorescent protein (GFP; Crameri., A., et al., Nat. Biotechnol. 14 (1996) 315-319) by using conventional cloning methods (Sambrook, Fritsch & Maniatis, Molecular Cloning, A Laboratory Manual, 3rd edition, CSHL Press, 2001). To create mutant sequences of the first ten codons of the wildtype sequence the following two sets of degenerated oligonucleotides were synthesised. The mutated sequences that were synthesised exploited the codon usage for each amino acid without changing the primary sequence. The bases that were changed are indicated in SEQ ID NO: 26 and SEQ ID NO: 27 using the following code: N = any base, Y = pyrimidine (C or T), R = purine (G or A), H = not G (i.e. A, T or C). Thus, two sets of forward primers were generated of which the respective consensus sequences are given in SEQ ID NO: 26 and SEQ ID NO: 27. Each set represented a mixture of primer molecules that essentially represented the possible combinations as defined by the bases that were changed.

In combination with the reverse primer according to SEQ ID NO: 28 that was selected to match an internal sequence of the GFP gene, a PCR reaction was made with the pIVEX-GFP WT Avitag (SEQ ID NO: 29) vector as template. Using the restriction enzymes XbaI and NcoI the PCR products were cleaved, firstly at the XbaI site in the forward primer and secondly at the NcoI site in the reverse primer. In parallel, the pIVEX-GFP WT Avitag vector was cleaved with the same restriction enzymes and the vector fragment was isolated.

Subsequently, the cleaved fragments were inserted into the pIVEX-GFP Avitag vector fragments.

The plasmids were ligated and subsequently transformed into a BL21 (DE 3) Lys S strain of E.coli (Novagen Inc., Madison, Wisconsin, USA) and plated out on LB medium with ampicillin (100 µg/ml) and IPTG (0.2 mM). After one day of growth bacterial colonies were screened under UV light for GFP expression. The colonies with the brightest fluorescence as judged by visual inspection were picked and plasmids from these colonies were isolated. The AviTag™-encoding DNA of these plasmids was subjected to sequence analysis. The screening procedure resulted in a number of mutant variants of the wildtype sequence encoding the AviTag™, whereby these variants stimulated a visibly increased GFP signal as compared to the signal of control transformants expressing the pIVEX-GFP WT Avitag vector.

The mutant variants of the wildtype sequence, i.e. DNA sequences encoding a polypeptide of SEQ ID NO: 1 capable of being biotinylated by holocarboxylase synthetase, are represented in SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, and SEQ ID NO: 24.

### Example 2

### Comparison of the mutant variants of the DNA sequence encoding the AviTag™ biotinylation polypeptide and the wildtype sequence

The wildtype sequence was compared with SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, and SEQ ID NO: 24, and a consensus sequence was derived for the mutant variants.

Accordingly, the consensus DNA sequence encoding SEQ ID NO: 1 was found to differ from the wildtype sequence, that is the sequence according to SEQ ID NO: 3, by nucleotide exchanges at 6 or more positions selected from the group consisting of the positions 4, 5, 6, 9, 10, 12, 15, 18, 21, 24 or 30.

Furthermore, the consensus DNA sequence was found to contain A or T at position 4, C or G at position 5, C, or T at position 6, A, C or T at position 9, C or T at position 10, A or G at position 12, C or T at position 15, C or T at position 18, C or T at position 21, C or T at position 24, and A or T at position 30. The consensus sequence is given in SEQ ID NO: 2.

Furthermore, between 5 and 11 nucleotides at said positions were found to be identical to the nucleotides at the same positions in SEQ ID NO: 4, with the proviso that all nucleotides at said positions were found to be identical to the nucleotides at the same positions in SEQ ID NO: 3 or SEQ ID NO: 4, or 10 nucleotides at said positions except position 9 were found to be identical to the nucleotides at the same positions in SEQ ID NO: 4 or SEQ ID NO: 3, and the nucleotide at position 9 was then found to be T.

### Example 3

### Construction of fusion polypeptides using a mutant variant of the DNA sequence encoding the AviTag™ biotinylation polypeptide

The mutated AviTag™ sequence according to SEQ ID NO: 12 was inserted in-frame in front of the chloramphenicol acetyl transferase (CAT) gene and the erythropoietin (EPO) gene by way of a PCR cloning approach analogous to the approach described in Example 1. As a result, the plasmids pIVEX-2.8 CAT mut Avitag and pIVEX-2.8 EPO mut Avitag were generated. In addition, the control plasmids pIVEX-2.8 CAT WT Avitag and pIVEX-2.8 EPO WT Avitag were generated that differed from pIVEX-2.8 CAT mut Avitag and pIVEX-2.8 EPO mut Avitag in that the wildtype AviTag™ sequence, i.e. SEQ ID NO: 3 replaced SEQ ID NO: 12.

All four of these plasmids, i.e. those containing the mutant variants as well as the wildtype controls, were then used for a polypeptide synthesis reaction using the RTS 500 HY Kit (Roche Diagnostics GmbH, Mannheim, Germany) as an in-vitro expression system. Each plasmid was used for a separate in-vitro expression. The polypeptide synthesis reactions were performed identically and in line with the instructions of the supplier. After the reactions were ended, 0.5 µl aliquots of each reaction mixture were directly applied on an SDS-PAGE gel. Another aliquot of each reaction was centrifuged for 15 min at 30,000 x g. The supernatants were removed and the pellet fractions were resuspended in the original volume of SDS sample buffer. Again 0.5 µl were applied on the same SDS Page gel.

After the run SDS gels were stained with Coomassie Brilliant Blue. Figure 1 shows the result. The fusion polypeptides encoded by the wildtype AviTag™ DNA sequence that was operably linked to the coding sequences of either CAT or EPO were present in smaller quantities as opposed to those fusion polypeptides in which the N-terminal tag was encoded by the mutated sequence of SEQ ID NO: 12. EPO in its unglycosylated form can be detected primarily in the pellet fraction. This result exemplifies, that a mutant variant of the DNA sequence encoding the AviTag™ biotinylation polypeptide, as compared to the wildtype sequence, enhances the formation of a fusion polypeptide, consisting of an N-terminal polypeptide according to SEQ ID NO: 1 and a C-terminal polypeptide with a biological function, by means of expression from a nucleic acid encoding said fusion polypeptide in a cell-free polypeptide synthesis reaction mixture.

### Example 4

### Quantification of expressed fusion polypeptides

The amounts of expressed fusion polypeptides were quantified by way of densitometric measurements of coomassie-stained bands in SDS gels that were obtained using the Lumi Imager F1 and the LumiAnalyst Software (Roche Diagnostics GmbH, Mannheim, Germany). Measurements were made according to the instructions of the manufacturer. Each analysed each gel contained control lanes in which defined amounts of marker proteins were electrophoresed in order to provide reference points for quantification. Table 1 provides results from the parallel experiments described in Example 3 and Figure 1.

**Table 1**

| **Quantification of fusion polypeptides expressed by the RTS 500 HY Kit using the expression vectors** | | | |
|---|---|---|---|
| Vector | SDS gel lane | Densitometric readout | Concentration [mg/ml] |
| pIVEX-2.8 CAT WT Avitag | 1 | 31.841 | 0.5 |
| pIVEX-2.8 CAT mut Avitag | 2 | 237.345 | 6.5 |
| pIVEX-2.8 EPO WT Avitag | 3 | 94.040 | 2.3 |
| pIVEX-2.8 EPO mut Avitag | 4 | 129.975 | 3.3 |
| pIVEX-2.8 CAT WT Avitag | 5 | 5.255 | 0 |
| pIVEX-2.8 CAT mut Avitag | 6 | 188.364 | 5.0 |
| pIVEX-2.8 EPO WT Avitag | 7 | 43.288 | 0.8 |
| pIVEX-2.8 EPO mut Avitag | 8 | 70.833 | 1.6 |

The results indicate that the mutant variant of the wildtype sequence as given in SEQ ID NO: 12 enhances the formation of the fusion polypeptide, consisting of an N-terminal polypeptide according to SEQ ID NO: 1 and a C-terminal polypeptide with a biological function in that at least 40% more fusion polypeptide is formed.

### List of References

Beckett, D., et al., Protein Sci. 8 (1999) 921-929
Chapman-Smith, A., and Cronan, J.E., Jr., J. Nutr. 129, 2S Suppl., (1999) 477S-484S
Crameri., A., et al., Nat. Biotechnol. 14 (1996) 315-319
Cronan, J.E., Jr., Cell 58 (1989) 427-429
Cronan, J.E., Jr., et al., J. Biol. Chem. 265 (1990) 10327-10333
de Boer, H.A., and Kastelein, R.A., In: Maximizing gene expression, Reznikoff, W.S., and Gold, L., (eds.), Butterworths, Boston, 1986, pp. 225-285
EP 0932664
Gu, J., et al., Biotechniques 17 (1994) 257, 260, 262
Kohanski, R.A., and Lane, M.D., Methods Enzymol. 184 (1990) 194-200
Makrides, S.C., Microbiol. Rev. 60 (1996) 512-538
Morag, E., et al., Anal. Biochem. 243 (1996) 257-263
Murtif, V.L., and Samols, D., J. Biol. Chem. 262 (1987) 11813-11816
Pratt et al., In: Transcription and Translation: A Practical Approach, Hames and Higgins (eds.), 1984, pp. 179-209, IRL Press
Pratt, J.M., et al., Nucleic Acids Research 9 (1981) 4459-4479
Sambrook, Fritsch & Maniatis, Molecular Cloning, A Laboratory Manual, 3rd edition, CSHL Press, 2001
Sano, T., and Cantor, C.R., Proc. Natl. Acad. Sci.USA 92 (1995) 3180-3184
Schatz, P.J., Biotechnology 11 (1993) 1138-1143
Spirin, A.S., et al., Science 242 (1988) 1162-1164
US 4,952,496
US 5,232,840
US 5,693,489
US 5,869,320
US 4,952,496
US 5,232,840
US 5,478,730
US 5,571,690
US 5,693,489
US 5,723,584
US 5,869,320
US 5,874,239
US 5,932,433
US 6,265,552
US 6,326,157
WO 00/55353
WO 00/58493
WO 95/04069
WO 99/37785
WO 99/50436
Zubay, G., Annu. Rev. Genet. 7 (1973) 267-287

## Claims

1. A nucleic acid of SEQ ID NO: 2 encoding a polypeptide of SEQ ID NO: 1 capable of being biotinylated by holocarboxylase synthetase,
**characterised in that**
said nucleic acid differs from SEQ ID NO: 3 by nucleotide exchanges at 6 or more positions selected from the group consisting of the positions 4, 5, 6, 9, 10, 12, 15, 18, 21, 24 or 30,
and
said nucleic acid, as compared to SEQ ID NO: 3, enhances the formation of a fusion polypeptide, consisting of an N-terminal polypeptide according to SEQ ID NO: 1 and a C-terminal polypeptide with a biological function, by means of expression from a nucleic acid encoding said fusion polypeptide in a cell-free polypeptide synthesis reaction mixture **in that** at least 40% more fusion polypeptide is formed,
whereby
the nucleic acid encoding said fusion polypeptide consists of a nucleic acid encoding said N-terminal polypeptide operably linked to a nucleic acid encoding said C-terminal polypeptide.

2. The nucleic acid according to claim 1
that is containing A or T at position 4, C or G at position 5, C, or T at position 6, A, C or T at position 9, C or T at position 10, A or G at position 12, C or T at position 15, C or T at position 18, C or T at position 21, C or T at position 24, and A or T at position 30,
**characterised in that**
between 5 and 11 nucleotides at said positions are identical to the nucleotides at the same positions in SEQ ID NO: 4,
with the proviso that
all nucleotides at said positions are identical to the nucleotides at the same positions in SEQ ID NO: 3 or SEQ ID NO: 4,
or
10 nucleotides at said positions except position 9 are identical to the nucleotides at the same positions in SEQ ID NO: 4 or SEQ ID NO: 3, and the nucleotide at position 9 is T.

3. A nucleic acid according to any of the claims 1 and 2,
**characterised in that**
the nucleic acid is selected from the group consisting of
SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23 or SEQ ID NO: 24.

4. An expression vector comprising a nucleic acid according to any of the claims 1 to 3.

5. A method of preparing a biotinylated polypeptide in a cell-free polypeptide synthesis reaction mixture which contains an RNA polymerase, ribosomes, tRNA, ATP, GTP, nucleotides and amino acids,
comprising the steps of
(a) forming in said reaction mixture a fusion polypeptide, consisting of an N-terminal polypeptide according to SEQ ID NO: 1 and a C-terminal polypeptide with a biological function, by means of expression from a nucleic acid consisting of a nucleic acid according to any of the claims 1 to 3 operably linked to a nucleic acid encoding the C-terminal polypeptide;
(b) biotinylating said fusion polypeptide in the presence of biotin and holocarboxylase synthetase;
(c) isolating said biotinylated fusion polypeptide from said mixture; or incubating said mixture with immobilised avidin or streptavidin under such conditions that said biotinylated fusion polypeptide is bound to said immobilised avidin or streptavidin.

6. The method according to claim 5,
**characterised in that**
the reaction mixture contains a nucleic acid encoding holocarboxylase synthetase according to SEQ ID NO: 25 that is expressed in said reaction mixture to provide holocarboxylase synthetase polypeptide.

7. Use of a nucleic acid according to any of the claims 1 to 3 for constructing, by way of genetic engineering, a nucleic acid encoding a fusion polypeptide, whereby the fusion polypeptide consists of an N-terminal polypeptide of SEQ ID NO: 1 and a C-terminal polypeptide with a biological function.

8. Use of a nucleic acid according to any of the claims 1 to 3 for expressing a fusion polypeptide, whereby the fusion polypeptide consists of an N-terminal polypeptide of SEQ ID NO: 1 and a C-terminal polypeptide with a biological function.

9. The use according to claim 8, **characterised in that** the fusion polypeptide is expressed in a cell-free polypeptide synthesis reaction mixture.

10. The use according to claim 8, **characterised in that** the fusion polypeptide is expressed in E.coli.


SEQUENCE LISTING

<110> Roche Diagnostics GmbH
      F. Hoffmann-La Roche AG

<120> Enhanced expression of fusion polypeptides with a
      biotinylation tag

<130> 21668 EP

<140>
<141>

<160> 31

<170> PatentIn Ver. 2.1

<210> 1
<211> 17
<212> PRT
<213> Artificial Sequence

<220>
<223> Description of Artificial Sequence:AviTagÖ
      biotinylation polypeptide, substrate for
      holoenzyme synthetase (BirA) of E.coli

<400> 1
Met Ser Gly Leu Asn Asp Ile Phe Glu Ala Gln Lys Ile Glu Trp His
  1               5                  10                  15

Glu

<210> 2
<211> 51
<212> DNA
<213> Artificial Sequence

<220>
<223> Description of Artificial Sequence:Consensus
      sequence for optimised nucleic acid sequences
      encoding the biotinylation polypeptide of SEQ ID
      NO: 1

<400> 2
atgwsygghy traaygayat yttygaggcw cagaaaatcg aatggcacga a          51

<210> 3
<211> 51
<212> DNA
<213> Artificial Sequence

<220>
<223> Description of Artificial Sequence:nucleic acid

sequence encoding the biotinylation polypeptide of
SEQ ID NO: 1; AviTagÖ wild-type sequence

<400> 3
atgtccggcc tgaacgacat cttcgaggct cagaaaatcg aatggcacga a          51


<210> 4
<211> 51
<212> DNA
<213> Artificial Sequence

<220>
<223> Description of Artificial Sequence:encoding the
      biotinylation polypeptide of SEQ ID NO: 1 for
      enhanced expression of biotinylated fusion
      polypeptides

<400> 4
atgagtggat taaatgatat ttttgaagca cagaaaatcg aatggcacga a          51


<210> 5
<211> 51
<212> DNA
<213> Artificial Sequence

<220>
<223> Description of Artificial Sequence:encoding the
      biotinylation polypeptide of SEQ ID NO: 1 for
      enhanced expression of biotinylated fusion
      polypeptides

<400> 5
atgagtggat taaatgatat tttcgaagca cagaaaatcg aatggcacga a          51


<210> 6
<211> 51
<212> DNA
<213> Artificial Sequence

<220>
<223> Description of Artificial Sequence:encoding the
      biotinylation polypeptide of SEQ ID NO: 1 for
      enhanced expression of biotinylated fusion
      polypeptides

<400> 6
atgagtggtt taaatgatat tttcgaggct cagaaaatcg aatggcacga a          51


<210> 7
<211> 51
<212> DNA
<213> Artificial Sequence

<220>
<223> Description of Artificial Sequence:encoding the
      biotinylation polypeptide of SEQ ID NO: 1 for
      enhanced expression of biotinylated fusion
      polypeptides

<400> 7
atgagtggct taaatgatat tttcgaggct cagaaaatcg aatggcacga a          51


<210> 8
<211> 51
<212> DNA
<213> Artificial Sequence

<220>
<223> Description of Artificial Sequence:encoding the
      biotinylation polypeptide of SEQ ID NO: 1 for
      enhanced expression of biotinylated fusion
      polypeptides

<400> 8
atgagtggtt taaatgatat cttcgaggct cagaaaatcg aatggcacga a          51


<210> 9
<211> 51
<212> DNA
<213> Artificial Sequence

<220>
<223> Description of Artificial Sequence:encoding the
      biotinylation polypeptide of SEQ ID NO: 1 for
      enhanced expression of biotinylated fusion
      polypeptides

<400> 9
atgagcggtt taaatgatat tttcgaggct cagaaaatcg aatggcacga a          51


<210> 10
<211> 51
<212> DNA
<213> Artificial Sequence

<220>
<223> Description of Artificial Sequence:encoding the
      biotinylation polypeptide of SEQ ID NO: 1 for
      enhanced expression of biotinylated fusion
      polypeptides

<400> 10
atgagtggtt taaatgacat tttcgaggct cagaaaatcg aatggcacga a          51

<210> 11
<211> 51
<212> DNA
<213> Artificial Sequence

<220>
<223> Description of Artificial Sequence:encoding the
      biotinylation polypeptide of SEQ ID NO: 1 for
      enhanced expression of biotinylated fusion
      polypeptides

<400> 11
atgagtggct taaatgatat cttcgaggct cagaaaatcg aatggcacga a          51


<210> 12
<211> 51
<212> DNA
<213> Artificial Sequence

<220>
<223> Description of Artificial Sequence:encoding the
      biotinylation polypeptide of SEQ ID NO: 1 for
      enhanced expression of biotinylated fusion
      polypeptides

<400> 12
atgagtggtt taaacgatat tttcgaggct cagaaaatcg aatggcacga a          51


<210> 13
<211> 51
<212> DNA
<213> Artificial Sequence

<220>
<223> Description of Artificial Sequence:encoding the
      biotinylation polypeptide of SEQ ID NO: 1 for
      enhanced expression of biotinylated fusion
      polypeptides

<400> 13
atgtctggtt taaatgatat tttcgaggct cagaaaatcg aatggcacga a          51


<210> 14
<211> 51
<212> DNA
<213> Artificial Sequence

<220>
<223> Description of Artificial Sequence:encoding the
      biotinylation polypeptide of SEQ ID NO: 1 for
      enhanced expression of biotinylated fusion
      polypeptides

<400> 14
atgagtggct taaatgacat tttcgaggct cagaaaatcg aatggcacga a          51


<210> 15
<211> 51
<212> DNA
<213> Artificial Sequence

<220>
<223> Description of Artificial Sequence:encoding the
      biotinylation polypeptide of SEQ ID NO: 1 for
      enhanced expression of biotinylated fusion
      polypeptides

<400> 15
atgagcggtt taaatgatat cttcgaggct cagaaaatcg aatggcacga a          51


<210> 16
<211> 51
<212> DNA
<213> Artificial Sequence

<220>
<223> Description of Artificial Sequence:encoding the
      biotinylation polypeptide of SEQ ID NO: 1 for
      enhanced expression of biotinylated fusion
      polypeptides

<400> 16
atgagcggtt taaacgatat tttcgaggct cagaaaatcg aatggcacga a          51


<210> 17
<211> 51
<212> DNA
<213> Artificial Sequence

<220>
<223> Description of Artificial Sequence:encoding the
      biotinylation polypeptide of SEQ ID NO: 1 for
      enhanced expression of biotinylated fusion
      polypeptides

<400> 17
atgagcggct taaatgatat tttcgaggct cagaaaatcg aatggcacga a          51


<210> 18
<211> 51
<212> DNA
<213> Artificial Sequence

<220>
<223> Description of Artificial Sequence:encoding the

biotinylation polypeptide of SEQ ID NO: 1 for
enhanced expression of biotinylated fusion
polypeptides

<400> 18
atgtctggtt taaatgatat cttcgaggct cagaaaatcg aatggcacga a 51

<210> 19
<211> 51
<212> DNA
<213> Artificial Sequence

<220>
<223> Description of Artificial Sequence:encoding the
biotinylation polypeptide of SEQ ID NO: 1 for
enhanced expression of biotinylated fusion
polypeptides

<400> 19
atgagtggtt taaacgatat cttcgaggct cagaaaatcg aatggcacga a 51

<210> 20
<211> 51
<212> DNA
<213> Artificial Sequence

<220>
<223> Description of Artificial Sequence:encoding the
biotinylation polypeptide of SEQ ID NO: 1 for
enhanced expression of biotinylated fusion
polypeptides

<400> 20
atgagcggct taaatgatat cttcgaggct cagaaaatcg aatggcacga a 51

<210> 21
<211> 51
<212> DNA
<213> Artificial Sequence

<220>
<223> Description of Artificial Sequence:encoding the
biotinylation polypeptide of SEQ ID NO: 1 for
enhanced expression of biotinylated fusion
polypeptides

<400> 21
atgagcggct taaatgatat cttcgaggct cagaaaatcg aatggcacga a 51

<210> 22
<211> 51
<212> DNA

<213> Artificial Sequence

<220>
<223> Description of Artificial Sequence:encoding the
      biotinylation polypeptide of SEQ ID NO: 1 for
      enhanced expression of biotinylated fusion
      polypeptides

<400> 22
atgagtggct taaacgatat tttcgaggct cagaaaatcg aatggcacga a          51


<210> 23
<211> 51
<212> DNA
<213> Artificial Sequence

<220>
<223> Description of Artificial Sequence:encoding the
      biotinylation polypeptide of SEQ ID NO: 1 for
      enhanced expression of biotinylated fusion
      polypeptides

<400> 23
atgagtggtt taaacgacat tttcgaggct cagaaaatcg aatggcacga a          51


<210> 24
<211> 51
<212> DNA
<213> Artificial Sequence

<220>
<223> Description of Artificial Sequence:encoding the
      biotinylation polypeptide of SEQ ID NO: 1 for
      enhanced expression of biotinylated fusion
      polypeptides

<400> 24
atgagtggct taaatgacat cttcgaggct cagaaaatcg aatggcacga a          51


<210> 25
<211> 966
<212> DNA
<213> Escherichia coli

<220>
<223> Coding sequence for biotin-holoenzyme synthetase;
      birA; according to GenBank entry gi:145430

<400> 25
atgaaggata acaccgtgcc actgaaattg attgccctgt tagcgaacgg tgaatttcac 60
tctggcgagc agttgggtga aacgctggga atgagccggg cggctattaa taaacacatt 120
cagacactgc gtgactgggg cgttgatgtc tttaccgttc cgggtaaagg atacagcctg 180
cctgagccta tccagttact taatgctaaa cagatattgg gtcagctgga tggcggtagt 240

gtagccgtgc tgccagtgat tgactccacg aatcagtacc ttcttgatcg tatcggagag 300
cttaaatcgg gcgatgcttg cattgcagaa taccagcagg ctggccgtgg tcgccggggt 360
cggaaatggt tttcgccttt tggcgcaaac ttatatttgt cgatgttctg gcgtctggaa 420
caaggcccgg cggcggcgat tggtttaagt ctggttatcg gtatcgtgat ggcggaagta 480
ttacgcaagc tgggtgcaga taaagttcgt gttaaatggc ctaatgacct ctatctgcag 540
gatcgcaagc tggcaggcat tctggtggag ctgactggca aaactggcga tgcggcgcaa 600
atagtcattg gagccgggat caacatggca atgcgccgtg ttgaagagag tgtcgttaat 660
caggggtgga tcacgctgca ggaagcgggg atcaatctcg atcgtaatac gttggcggcc 720
atgctaatac gtgaattacg tgctgcgttg gaactcttcg aacaagaagg attggcacct 780
tatctgtcgc gctgggaaaa gctggataat tttattaatc gcccagtgaa acttatcatt 840
ggtgataaag aaatatttgg catttcacgc ggaatagaca aacagggggc tttattactt 900
gagcaggatg gaataataaa accctggatg ggcggtgaaa tatccctgcg tagtgcagaa 960
aaataa 966

<210> 26
<211> 92
<212> DNA
<213> Artificial Sequence

<220>
<223> Description of Artificial Sequence:forward primer

<400> 26
gtttccctct agaaataatt ttgtttaact ttaagaagga gatataccat gtcnggnytn 60
aaygayatht tygargcnca gaaaatcgaa tg 92

<210> 27
<211> 92
<212> DNA
<213> Artificial Sequence

<220>
<223> Description of Artificial Sequence:forward primer

<400> 27
gtttccctct agaaataatt ttgtttaact ttaagaagga gatataccat gagyggnytn 60
aaygayatht tygargcnca gaaaatcgaa tg 92

<210> 28
<211> 30
<212> DNA
<213> Artificial Sequence

<220>
<223> Description of Artificial Sequence:reverse primer

<400> 28
caagtgttgg ccatggaaca ggtagttttc 30

<210> 29
<211> 8572
<212> DNA

<213> Artificial Sequence

<220>
<223> Description of Artificial Sequence:pIVEX-GFP WT
      Avitag; GFP vector with coding sequence for
      wildtype AviTag-GFP fusion polypeptide

<400> 29
tcgcgcgttt cggtgatgac ggtgaaaacc tctgacacat gcagctcccg agcgcgcaaa 60
gccactactg ccacttttgg agactgtgta cgtcgagggc gagacggtca cagcttgtct 120
gtaagcggat gccgggagca gacaagcccg ctctgccagt gtcgaacaga cattcgccta 180
cggccctcgt ctgttcgggc tcagggcgcg tcagcgggtg ttggcgggtg tcggggctgg 240
cttaactatg agtcccgcgc agtcgcccac aaccgcccac agccccgacc gaattgatac 300
cggcatcaga gcagattgta ctgagagtgc accatatgcg gtgtgaaata gccgtagtct 360
cgtctaacat gactctcacg tggtatacgc cacactttat ccgcacagat gcgtaaggag 420
aaaataccgc atcaggcgcc attcgccatt ggcgtgtcta cgcattcctc ttttatggcg 480
tagtccgcgg taagcggtaa caggctgcgc aactgttggg aagggcgatc ggtgcgggcc 540
tcttcgctat gtccgacgcg ttgacaaccc ttcccgctag ccacgcccgg agaagcgata 600
tacgccagct ggcgaaaggg ggatgtgctg caaggcgatt aagttgggta atgcggtcga 660
ccgctttccc cctacacgac gttccgctaa ttcaacccat acgccagggt tttcccagtc 720
acgacgttgt aaaacgacgg ccagtgccaa tgcggtccca aaagggtcag tgctgcaaca 780
ttttgctgcc ggtcacggtt gcttgcatgc aaggagatgg cgcccaacag tcccccggcc 840
acggggcctg cgaacgtacg ttcctctacc gcgggttgtc agggggccgg tgccccggac 900
ccaccatacc cacgccgaaa caagcgctca tgagcccgaa gtggcgagcc ggtggtatgg 960
gtgcggcttt gttcgcgagt actcgggctt caccgctcgg cgatcttccc catcggtgat 1020
gtcggcgata taggcgccag caaccgcacc gctagaaggg gtagccacta cagccgctat 1080
atccgcggtc gttggcgtgg tgtggcgccg gtgatgccgg ccacgatgcg tccggcgtag 1140
aggatcgaga acaccgcggc cactacggcc ggtgctacgc aggccgcatc tcctagctct 1200
tctcgatccc gcgaaattaa tacgactcac tatagggaga ccacaacggt agagctaggg 1260
cgctttaatt atgctgagtg atatccctct ggtgttgcca ttccctctag aaataatttt 1320
gtttaacttt aagaaggaga tataccatgt aagggagatc tttattaaaa caaattgaaa 1380
ttcttcctct atatggtaca ccggcctgaa cgacatcttc gaggctcaga aaatcgaatg 1440
gcacgaaact ggccggactt gctgtagaag ctccgagtct tttagcttac cgtgctttga 1500
agcaaaggag aagaactttt cactggagtt gtcccaattc ttgttgaatt tcgtttcctc 1560
ttcttgaaaa gtgacctcaa cagggttaag aacaacttaa agatggtgat gttaatgggc 1620
acaaattttc tgtcagtgga gagggtgaag tctaccacta caattacccg tgtttaaaag 1680
acagtcacct ctcccacttc gtgatgctac atacggaaag cttaccctta aatttatttg 1740
cactactgga cactacgatg tatgcctttc gaatgggaat ttaaataaac gtgatgacct 1800
aaactacctg ttccatggcc aacacttgtc actactttct cttatggtgt tttgatggac 1860
aaggtaccgg ttgtgaacag tgatgaaaga gaataccaca tcaatgcttt tcccgttatc 1920
cggatcatat gaaacggcat gactttttca agttacgaaa agggcaatag gcctagtata 1980
ctttgccgta ctgaaaaagt agagtgccat gcccgaaggt tatgtacagg aacgcactat 2040
atctttcaaa tctcacggta cgggcttcca atacatgtcc ttgcgtgata tagaaagttt 2100
gatgacggga actacaagac gcgtgctgaa gtcaagtttg aaggtgatac ctactgccct 2160
tgatgttctg cgcacgactt cagttcaaac ttccactatg ccttgttaat cgtatcgagt 2220
taaaaggtat tgattttaaa gaagatggaa ggaacaatta gcatagctca attttccata 2280
actaaaattt cttctacctt acattctcgg acacaaactc gagtacaact ataactcaca 2340
caatgtatac tgtaagagcc tgtgtttgag ctcatgttga tattgagtgt gttacatatg 2400
atcacggcag acaaacaaaa gaatggaatc aaagctaact tcaaaattcg tagtgccgtc 2460
tgtttgtttt cttaccttag tttcgattga agttttaagc ccacaacatt gaagatggat 2520
ccgttcaact agcagaccat tatcaacaaa ggtgttgtaa cttctaccta ggcaagttga 2580
tcgtctggta atagttgttt atactccaat tggcgatggc cctgtccttt taccagacaa 2640
ccattacctg tatgaggtta accgctaccg ggacaggaaa atggtctgtt ggtaatggac 2700
tcgacacaat ctgccctttc gaaagatccc aacgaaaaga gagaccacat agctgtgtta 2760
gacgggaaag ctttctaggg ttgcttttct ctctggtgta ggtccttctt gagtttgtaa 2820
cagctgctgg gattacacat ggcatggatg ccaggaagaa ctcaaacatt gtcgacgacc 2880

ctaatgtgta ccgtacctac aactatacaa acccgggggg ggttctcatc atcatcatca 2940
tcattaataa ttgatatgtt tgggcccccc ccaagagtag tagtagtagt agtaattatt 3000
aagggcgaat tccagcacac tggcggccgt tactagtgga tccggctgct ttcccgctta 3060
aggtcgtgtg accgccggca atgatcacct aggccgacga aacaaagccc gaaaggaagc 3120
tgagttggct gctgccaccg ctgagcaata ttgtttcggg ctttccttcg actcaaccga 3180
cgacggtggc gactcgttat actagcataa ccccttgggg cctctaaacg ggtcttgagg 3240
ggttttttgc tgatcgtatt ggggaacccc ggagatttgc ccagaactcc ccaaaaaacg 3300
tgaaaggagg aactatatcc ggatatccac aggacgggtg tggtcgccat actttcctcc 3360
ttgatatagg cctataggtg tcctgcccac accagcggta gatcgcgtag tcgatagtgg 3420
ctccaagtag cgaagcgagc aggactgggc ctagcgcatc agctatcacc gaggttcatc 3480
gcttcgctcg tcctgacccg ggcggccaaa gcggtcggac agtgctccga gaacgggtgc 3540
gcatagaaat ccgccggttt cgccagcctg tcacgaggct cttgcccacg cgtatcttta 3600
tgcatcaacg catatagcgc tagcagcacg ccatagtgac tggcgatgct acgtagttgc 3660
gtatatcgcg atcgtcgtgc ggtatcactg accgctacga gtcggaatgg acgatatccc 3720
gcaagaggcc cggcagtacc ggcataacca cagccttacc tgctataggg cgttctccgg 3780
gccgtcatgg ccgtattggt agcctatgcc tacagcatcc agggtgacgg tgccgaggat 3840
gacgatgagc tcggatacgg atgtcgtagg tcccactgcc acggctccta ctgctactcg 3900
gcattgttag atttcataca cggtgcctga ctgcgttagc aatttaactg cgtaacaatc 3960
taaagtatgt gccacggact gacgcaatcg ttaaattgac tgataaacta ccgcattaaa 4020
gcttatcgat gataagctgt caaacatgag actatttgat ggcgtaattt cgaatagcta 4080
ctattcgaca gtttgtactc aattcgtaat catggtcata gctgtttcct gtgtgaaatt 4140
gttatccgct ttaagcatta gtaccagtat cgacaaagga cacactttaa caataggcga 4200
cacaattcca cacaacatac gagccggaag cataaagtgt aaagcctggg gtgttaaggt 4260
gtgttgtatg ctcggccttc gtatttcaca tttcggaccc gtgcctaatg agtgagctaa 4320
ctcacattaa ttgcgttgcg ctcactgccc cacggattac tcactcgatt gagtgtaatt 4380
aacgcaacgc gagtgacggg gctttccagt cgggaaacct gtcgtgccag ctgcattaat 4440
gaatcggcca cgaaaggtca gccctttgga cagcacggtc gacgtaatta cttagccggt 4500
acgcgcgggg agaggcggtt tgcgtattgg gcgctcttcc gcttcctcgc tgcgcgcccc 4560
tctccgccaa acgcataacc cgcgagaagg cgaaggagcg tcactgactc gctgcgctcg 4620
gtcgttcggc tgcggcgagc ggtatcagct agtgactgag cgacgcgagc cagcaagccg 4680
acgccgctcg ccatagtcga cactcaaagg cggtaatacg gttatccaca gaatcagggg 4740
ataacgcagg gtgagtttcc gccattatgc caataggtgt cttagtcccc tattgcgtcc 4800
aaagaacatg tgagcaaaag gccagcaaaa ggccaggaac cgtaaaaagg tttcttgtac 4860
actcgttttc cggtcgtttt ccggtccttg gcatttttcc ccgcgttgct ggcgtttttc 4920
cataggctcc gcccccctga cgagcatcac ggcgcaacga ccgcaaaaag gtatccgagg 4980
cggggggact gctcgtagtg aaaaatcgac gctcaagtca gaggtggcga aacccgacag 5040
gactataaag tttttagctg cgagttcagt ctccaccgct ttgggctgtc ctgatatttc 5100
ataccaggcg tttccccctg gaagctccct cgtgcgctct cctgttccga tatggtccgc 5160
aaagggggac cttcgaggga gcacgcgaga ggacaaggct ccctgccgct taccggatac 5220
ctgtccgcct ttctcccttc gggaagcgtg gggacggcga atggcctatg gacaggcgga 5280
aagagggaag cccttcgcac gcgctttctc atagctcacg ctgtaggtat ctcagttcgg 5340
tgtaggtcgt cgcgaaagag tatcgagtgc gacatccata gagtcaagcc acatccagca 5400
tcgctccaag ctgggctgtg tgcacgaacc ccccgttcag cccgaccgct agcgaggttc 5460
gacccgacac acgtgcttgg ggggcaagtc gggctggcga gcgccttatc cggtaactat 5520
cgtcttgagt ccaacccggt aagacacgac cgcggaatag gccattgata gcagaactca 5580
ggttgggcca ttctgtgctg ttatcgccac tggcagcagc cactggtaac aggattagca 5640
gagcgaggta aatagcggtg accgtcgtcg gtgaccattg tcctaatcgt ctcgctccat 5700
tgtaggcggt gctacagagt tcttgaagtg gtggcctaac tacggctaca acatccgcca 5760
cgatgtctca agaacttcac caccggattg atgccgatgt ctagaaggac agtatttggt 5820
atctgcgctc tgctgaagcc agttaccttc gatcttcctg tcataaacca tagacgcgag 5880
acgacttcgg tcaatggaag ggaaaaagag ttggtagctc ttgatccggc aaacaaacca 5940
ccgctggtag cctttttctc aaccatcgag aactaggccg tttgtttggt ggcgaccatc 6000
cggtggtttt tttgtttgca agcagcagat tacgcgcaga aaaaaaggat gccaccaaaa 6060
aaacaaacgt tcgtcgtcta atgcgcgtct tttttcccta ctcaagaaga tcctttgatc 6120
ttttctacgg ggtctgacgc tcagtggaac gagttcttct aggaaactag aaaagatgcc 6180
ccagactgcg agtcaccttg gaaaactcac gttaagggat tttggtcatg agattatcaa 6240

aaaggatctt cttttgagtg caattcccta aaaccagtac tctaatagtt tttcctagaa 6300
cacctagatc cttttaaatt aaaaatgaag ttttaaatca atctaaagta gtggatctag 6360
gaaaatttaa tttttacttc aaaatttagt tagatttcat tatatgagta aacttggtct 6420
gacagttacc aatgcttaat cagtgaggca atatactcat ttgaaccaga ctgtcaatgg 6480
ttacgaatta gtcactccgt cctatctcag cgatctgtct atttcgttca tccatagttg 6540
cctgactccc ggatagagtc gctagacaga taaagcaagt aggtatcaac ggactgaggg 6600
cgtcgtgtag ataactacga tacgggaggg cttaccatct ggccccagtg gcagcacatc 6660
tattgatgct atgccctccc gaatggtaga ccggggtcac ctgcaatgat accgcgagac 6720
ccacgctcac cggctccaga tttatcagca gacgttacta tggcgctctg ggtgcgagtg 6780
gccgaggtct aaatagtcgt ataaaccagc cagccggaag ggccgagcgc agaagtggtc 6840
ctgcaacttt tatttggtcg gtcggccttc ccggctcgcg tcttcaccag gacgttgaaa 6900
atccgcctcc atccagtcta ttaattgttg ccgggaagct agagtaagta taggcggagg 6960
taggtcagat aattaacaac ggcccttcga tctcattcat gttcgccagt taatagtttg 7020
cgcaacgttg ttgccattgc tacaggcatc caagcggtca attatcaaac gcgttgcaac 7080
aacggtaacg atgtccgtag gtggtgtcac gctcgtcgtt tggtatggct tcattcagct 7140
ccggttccca caccacagtg cgagcagcaa accataccga agtaagtcga ggccaagggt 7200
acgatcaagg cgagttacat gatcccccat gttgtgcaaa aaagcggtta tgctagttcc 7260
gctcaatgta ctagggggta caacacgttt tttcgccaat gctccttcgg tcctccgatc 7320
gttgtcagaa gtaagttggc cgcagtgtta cgaggaagcc aggaggctag caacagtctt 7380
cattcaaccg gcgtcacaat tcactcatgg ttatggcagc actgcataat tctcttactg 7440
tcatgccatc agtgagtacc aataccgtcg tgacgtatta agagaatgac agtacggtag 7500
cgtaagatgc ttttctgtga ctggtgagta ctcaaccaag tcattctgag gcattctacg 7560
aaaagacact gaccactcat gagttggttc agtaagactc aatagtgtat gcggcgaccg 7620
agttgctctt gcccggcgtc aatacgggat ttatcacata cgccgctggc tcaacgagaa 7680
cgggccgcag ttatgcccta aataccgcgc cacatagcag aactttaaaa gtgctcatca 7740
ttggaaaacg ttatggcgcg gtgtatcgtc ttgaaatttt cacgagtagt aaccttttgc 7800
ttcttcgggg cgaaaactct caaggatctt accgctgttg agatccagtt aagaagcccc 7860
gcttttgaga gttcctagaa tggcgacaac tctaggtcaa cgatgtaacc cactcgtgca 7920
cccaactgat cttcagcatc ttttactttc gctacattgg gtgagcacgt gggttgacta 7980
gaagtcgtag aaaatgaaag accagcgttt ctgggtgagc aaaaacagga aggcaaaatg 8040
ccgcaaaaaa tggtcgcaaa gacccactcg tttttgtcct tccgttttac ggcgtttttt 8100
gggaataagg gcgacacgga aatgttgaat actcatactc ttcctttttc cccttattcc 8160
cgctgtgcct ttacaactta tgagtatgag aaggaaaaag aatattattg aagcatttat 8220
cagggttatt gtctcatgag cggatacata ttataataac ttcgtaaata gtcccaataa 8280
cagagtactc gcctatgtat tttgaatgta tttagaaaaa taaacaaata ggggttccgc 8340
gcacatttcc aaacttacat aaatcttttt atttgtttat ccccaaggcg cgtgtaaagg 8400
ccgaaaagtg ccacctgacg tctaagaaac cattattatc atgacattaa ggcttttcac 8460
ggtggactgc agattctttg gtaataatag tactgtaatt cctataaaaa taggcgtatc 8520
acgaggccct ttcgtcggat atttttatcc gcatagtgct ccgggaaagc ag 8572

<210> 30
<211> 8408
<212> DNA
<213> Artificial Sequence

<220>
<223> Description of Artificial Sequence:pIVEX-2.8 CAT
      mut Avitag; vector with coding sequence for
      mutated AviTag fused to chloramphenicol acetyl
      transferase (CAT)

<400> 30
tcgcgcgttt cggtgatgac ggtgaaaacc tctgacacat gcagctcccg agcgcgcaaa 60
gccactactg ccacttttgg agactgtgta cgtcgagggc gagacggtca cagcttgtct 120
gtaagcggat gccgggagca gacaagcccg ctctgccagt gtcgaacaga cattcgccta 180

cggccctcgt ctgttcgggc tcagggcgcg tcagcgggtg ttggcgggtg tcggggctgg 240
cttaactatg agtcccgcgc agtcgcccac aaccgcccac agccccgacc gaattgatac 300
cggcatcaga gcagattgta ctgagagtgc accatatatg cggtgtgaaa gccgtagtct 360
cgtctaacat gactctcacg tggtatatac gccacacttt taccgcacag atgcgtaagg 420
agaaaatacc gcatcaggcg ccattcgcca atggcgtgtc tacgcattcc tcttttatgg 480
cgtagtccgc ggtaagcggt ttcaggctgc gcaactgttg ggaagggcga tcggtgcggg 540
cctcttcgct aagtccgacg cgttgacaac ccttcccgct agccacgccc ggagaagcga 600
attacgccag ctggcgaaag ggggatgtgc tgcaaggcga ttaagttggg taatgcggtc 660
gaccgctttc cccctacacg acgttccgct aattcaaccc taacgccagg gttttcccag 720
tcacgacgtt gtaaaacgac ggccagtgcc attgcggtcc caaaagggtc agtgctgcaa 780
cattttgctg ccggtcacgg aagcttgcat gcaaggagat ggcgcccaac agtcccccgg 840
ccacggggcc ttcgaacgta cgttcctcta ccgcgggttg tcagggggcc ggtgccccgg 900
tgccaccata cccacgccga aacaagcgct catgagcccg aagtggcgag acggtggtat 960
gggtgcggct ttgttcgcga gtactcgggc ttcaccgctc cccgatcttc cccatcggtg 1020
atgtcggcga tataggcgcc agcaaccgca gggctagaag gggtagccac tacagccgct 1080
atatccgcgg tcgttggcgt cctgtggcgc cggtgatgcc ggccacgatg cgtccggcgt 1140
agaggatcga ggacaccgcg gccactacgg ccggtgctac gcaggccgca tctcctagct 1200
gatctcgatc ccgcgaaatt aatacgactc actataggga gaccacaacg ctagagctag 1260
ggcgctttaa ttatgctgag tgatatccct ctggtgttgc gtttccctct agaaataatt 1320
ttgtttaact ttaagaagga gatataccat caaaggagaa tctttattaa aacaaattga 1380
aattcttcct ctatatggta gagtggttta aacgatattt tcgaggctca gaaaatcgaa 1440
tggcacgaaa ctcaccaaat ttgctataaa agctccgagt cttttagctt accgtgcttt 1500
tcgaaggccg cggccgctta attaaacata tgaccatgga gaaaaaaatc agcttccggc 1560
gccggcgaat taatttgtat actggtacct ctttttttag actggatata ccaccgttga 1620
tatatcccaa tggcatcgta aagaacattt tgacctatat ggtggcaact atatagggtt 1680
accgtagcat ttcttgtaaa tgaggcattt cagtcagttg ctcaatgtac ctataaccag 1740
accgttcagc actccgtaaa gtcagtcaac gagttacatg gatattggtc tggcaagtcg 1800
tggatattac ggccttttta aagaccgtaa agaaaaataa gcacaagttt acctataatg 1860
ccggaaaaat ttctggcatt tctttttatt cgtgttcaaa tatccggcct ttattcacat 1920
tcttgcccgc ctgatgaatg ctcatccgga ataggccgga aataagtgta agaacgggcg 1980
gactacttac gagtaggcct actccgtatg gcaatgaaag acggtgagct ggtgatatgg 2040
gatagtgttc tgaggcatac cgttactttc tgccactcga ccactatacc ctatcacaag 2100
acccttgtta caccgttttc catgagcaaa ctgaaacgtt ttcatcgctc tgggaacaat 2160
gtggcaaaag gtactcgttt gactttgcaa aagtagcgag tggagtgaat accacgacga 2220
tttccggcag tttctacaca tatattcgca acctcactta tggtgctgct aaaggccgtc 2280
aaagatgtgt atataagcgt agatgtggcg tgttacggtg aaaacctggc ctatttccct 2340
aaagggttta tctacaccgc acaatgccac ttttggaccg gataaaggga tttcccaaat 2400
ttgagaatat gtttttcgtc tcagccaatc cctgggtgag tttcaccagt aactcttata 2460
caaaaagcag agtcggttag ggacccactc aaagtggtca tttgatttaa acgtggccaa 2520
tatggacaac ttcttcgccc ccgttttcac aaactaaatt tgcaccggtt atacctgttg 2580
aagaagcggg ggcaaaagtg gatgggcaaa tattatacgc aaggcgacaa ggtgctgatg 2640
ccgctggcga ctacccgttt ataatatgcg ttccgctgtt ccacgactac ggcgaccgct 2700
ttcaggttca tcatgccgtt tgtgatggct tccatgtcgg cagaatgctt aagtccaagt 2760
agtacggcaa acactaccga aggtacagcc gtcttacgaa aatgaattac aacagtactg 2820
cgatgagtgg cagggcgggg cgcccgggat ttacttaatg ttgtcatgac gctactcacc 2880
gtcccgcccc gcgggcccta ccggtaagat ccggctgcta acaaagcccg aaaggaagct 2940
gagttggctg ggccattcta ggccgacgat tgtttcgggc tttccttcga ctcaaccgac 3000
ctgccaccgc tgagcaataa ctagcataac cccttggggc ctctaaacgg gacggtggcg 3060
actcgttatt gatcgtattg gggaaccccg gagatttgcc gtcttgaggg gttttttgct 3120
gaaaggagga actatatccg gatatccaca cagaactccc caaaaaacga ctttcctcct 3180
tgatataggc ctataggtgt ggacgggtgt ggtcgccatg atcgcgtagt cgatagtggc 3240
tccaagtagc cctgcccaca ccagcggtac tagcgcatca gctatcaccg aggttcatcg 3300
gaagcgagca ggactgggcg gcggccaaag cggtcggaca gtgctccgag cttcgctcgt 3360
cctgacccgc cgccggtttc gccagcctgt cacgaggctc aacgggtgcg catagaaatt 3420
gcatcaacgc atatagcgct agcagcacgc ttgcccacgc gtatctttaa cgtagttgcg 3480
tatatcgcga tcgtcgtgcg catagtgact ggcgatgctg tcggaatgga cgatatcccg 3540

caagaggccc gtatcactga ccgctacgac agccttacct gctatagggc gttctccggg 3600
ggcagtaccg gcataaccaa gcctatgcct acagcatcca gggtgacggt ccgtcatggc 3660
cgtattggtt cggatacgga tgtcgtaggt cccactgcca gccgaggatg acgatgagcg 3720
cattgttaga tttcatacac ggtgcctgac cggctcctac tgctactcgc gtaacaatct 3780
aaagtatgtg ccacggactg tgcgttagca atttaactgt gataaactac cgcattaaag 3840
cttatcgatg acgcaatcgt taaattgaca ctatttgatg gcgtaatttc gaatagctac 3900
ataagctgtc aaacatgaga attcgtaatc atgtcatagc tgtttcctgt tattcgacag 3960
tttgtactct taagcattag tacagtatcg acaaaggaca gtgaaattgt tatccgctca 4020
caattccaca caacatacga gccggaagca cactttaaca ataggcgagt gttaaggtgt 4080
gttgtatgct cggccttcgt taaagtgtaa agcctggggt gcctaatgag tgagctaact 4140
cacattaatt atttcacatt tcggacccca cggattactc actcgattga gtgtaattaa 4200
gcgttgcgct cactgcccgc tttccagtcg ggaaacctgt cgtgccagct cgcaacgcga 4260
gtgacgggcg aaaggtcagc cctttggaca gcacggtcga gcattaatga atcggccaac 4320
gcgcggggag aggcggtttg cgtattgggc cgtaattact tagccggttg cgcgcccctc 4380
tccgccaaac gcataacccg gctcttccgc ttcctcgctc actgactcgc tgcgctcggt 4440
cgttcggctg cgagaaggcg aaggagcgag tgactgagcg acgcgagcca gcaagccgac 4500
cggcgagcgg tatcagctca ctcaaaggcg gtaatacggt tatccacaga gccgctcgcc 4560
atagtcgagt gagtttccgc cattatgcca ataggtgtct atcaggggat aacgcaggaa 4620
agaacatgtg agcaaaaggc cagcaaaagg tagtccccta ttgcgtcctt tcttgtacac 4680
tcgttttccg gtcgttttcc ccaggaaccg taaaaaggcc gcgttgctgg cgtttttcca 4740
taggctccgc ggtccttggc atttttccgg cgcaacgacc gcaaaaaggt atccgaggcg 4800
ccccctgacg agcatcacaa aaatcgacgc tcaagtcaga ggtggcgaaa gggggactgc 4860
tcgtagtgtt tttagctgcg agttcagtct ccaccgcttt cccgacagga ctataaagat 4920
accaggcgtt tccccctgga agctccctcg gggctgtcct gatatttcta tggtccgcaa 4980
agggggacct tcgagggagc tgcgctctcc tgttccgacc ctgccgctta ccggatacct 5040
gtccgccttt acgcgagagg acaaggctgg gacggcgaat ggcctatgga caggcggaaa 5100
ctcccttcgg gaagcgtggc gctttctcat agctcacgct gtaggtatct gagggaagcc 5160
cttcgcaccg cgaaagagta tcgagtgcga catccataga cagttcggtg taggtcgttc 5220
gctccaagct gggctgtgtg cacgaacccc gtcaagccac atccagcaag cgaggttcga 5280
cccgacacac gtgcttgggg ccgttcagcc cgaccgctgc gccttatccg gtaactatcg 5340
tcttgagtcc ggcaagtcgg gctggcgacg cggaataggc cattgatagc agaactcagg 5400
aacccggtaa gacacgactt atcgccactg gcagcagcca ctggtaacag ttgggccatt 5460
ctgtgctgaa tagcggtgac cgtcgtcggt gaccattgtc gattagcaga gcgaggtatg 5520
taggcggtgc tacagagttc ttgaagtggt ctaatcgtct cgctccatac atccgccacg 5580
atgtctcaag aacttcacca ggcctaacta cggctacact agaaggacag tatttggtat 5640
ctgcgctctg ccggattgat gccgatgtga tcttcctgtc ataaaccata gacgcgagac 5700
ctgaagccag ttaccttcgg aaaaagagtt ggtagctctt gatccggcaa gacttcggtc 5760
aatggaagcc tttttctcaa ccatcgagaa ctaggccgtt acaaaccacc gctggtagcg 5820
gtggtttttt tgtttgcaag cagcagatta tgtttggtgg cgaccatcgc caccaaaaaa 5880
acaaacgttc gtcgtctaat cgcgcagaaa aaaaggatct caagaagatc ctttgatctt 5940
ttctacgggg gcgcgtcttt ttttcctaga gttcttctag gaaactagaa aagatgcccc 6000
tctgacgctc agtggaacga aaactcacgt taagggattt tggtcatgag agactgcgag 6060
tcaccttgct tttgagtgca attccctaaa accagtactc attatcaaaa aggatcttca 6120
cctagatcct tttaaattaa aaatgaagtt taatagtttt tcctagaagt ggatctagga 6180
aaatttaatt tttacttcaa ttaaatcaat ctaaagtata tatgagtaaa cttggtctga 6240
cagttaccaa aatttagtta gatttcatat atactcattt gaaccagact gtcaatggtt 6300
tgcttaatca gtgaggcacc tatctcagcg atctgtctat ttcgttcatc acgaattagt 6360
cactccgtgg atagagtcgc tagacagata aagcaagtag catagttgcc tgactccccg 6420
tcgtgtagat aactacgata cgggagggct gtatcaacgg actgaggggc agcacatcta 6480
ttgatgctat gccctcccga taccatctgg ccccagtgct gcaatgatac cgcgagaccc 6540
acgctcaccg atggtagacc ggggtcacga cgttactatg gcgctctggg tgcgagtggc 6600
gctccagatt tatcagcaat aaaccagcca gccggaaggg ccgagcgcag cgaggtctaa 6660
atagtcgtta tttggtcggt cggccttccc ggctcgcgtc aagtggtcct gcaactttat 6720
ccgcctccat ccagtctatt aattgttgcc ttcaccagga cgttgaaata ggcggaggta 6780
ggtcagataa ttaacaacgg gggaagctag agtaagtagt tcgccagtta atagtttgcg 6840
caacgttgtt cccttcgatc tcattcatca agcggtcaat tatcaaacgc gttgcaacaa 6900

gccattgcta caggcatcgt ggtgtcacgc tcgtcgtttg gtatggcttc cggtaacgat 6960
gtccgtagca ccacagtgcg agcagcaaac cataccgaag attcagctcc ggttcccaac 7020
gatcaaggcg agttacatga tcccccatgt taagtcgagg ccaagggttg ctagttccgc 7080
tcaatgtact agggggtaca tgtgcaaaaa agcggttagc tccttcggtc ctccgatcgt 7140
tgtcagaagt acacgttttt tcgccaatcg aggaagccag gaggctagca acagtcttca 7200
aagttggccg cagtgttatc actcatggtt atggcagcac tgcataattc ttcaaccggc 7260
gtcacaatag tgagtaccaa taccgtcgtg acgtattaag tcttactgtc atgccatccg 7320
taagatgctt ttctgtgact ggtgagtact agaatgacag tacggtaggc attctacgaa 7380
aagacactga ccactcatga caaccaagtc attctgagaa tagtgtatgc ggcgaccgag 7440
ttgctcttgc gttggttcag taagactctt atcacatacg ccgctggctc aacgagaacg 7500
ccggcgtcaa tacgggataa taccgcgcca catagcagaa ctttaaaagt ggccgcagtt 7560
atgccctatt atggcgcggt gtatcgtctt gaaattttca gctcatcatt ggaaaacgtt 7620
cttcggggcg aaaactctca aggatcttac cgagtagtaa ccttttgcaa gaagccccgc 7680
ttttgagagt tcctagaatg cgctgttgag atccagttcg atgtaaccca ctcgtgcacc 7740
caactgatct gcgacaactc taggtcaagc tacattgggt gagcacgtgg gttgactaga 7800
tcagcatctt ttactttcac cagcgtttct gggtgagcaa aaacaggaag agtcgtagaa 7860
aatgaaagtg gtcgcaaaga cccactcgtt tttgtccttc gcaaaatgcc gcaaaaaagg 7920
gaataagggc gacacggaaa tgttgaatac cgttttacgg cgttttttcc cttattcccg 7980
ctgtgccttt acaacttatg tcatactctt cctttttcaa tattattgaa gcatttatca 8040
gggttattgt agtatgagaa ggaaaaagtt ataataactt cgtaaatagt cccaataaca 8100
ctcatgagcg gatacatatt tgaatgtatt tagaaaaata aacaaatagg gagtactcgc 8160
ctatgtataa acttacataa atctttttat ttgtttatcc ggttccgcgc acatttcccc 8220
gaaaagtgcc acctgacgtc taagaaacca ccaaggcgcg tgtaaagggg cttttcacgg 8280
tggactgcag attctttggt ttattatcat gacattaacc tataaaaata ggcgtatcac 8340
gaggcccttt aataatagta ctgtaattgg atatttttat ccgcatagtg ctccgggaaa 8400
cgtcgcag 8408

<210> 31
<211> 8138
<212> DNA
<213> Artificial Sequence

<220>
<223> Description of Artificial Sequence:pIVEX-2.8 EPO
      mut Avitag; vector with coding sequence for
      mutated AviTag fused to erythropoetin (EPO)

<400> 31
tcgcgcgttt cggtgatgac ggtgaaaacc tctgacacat gcagctcccg agcgcgcaaa 60
gccactactg ccacttttgg agactgtgta cgtcgagggc gagacggtca cagcttgtct 120
gtaagcggat gccgggagca gacaagcccg ctctgccagt gtcgaacaga cattcgccta 180
cggccctcgt ctgttcgggc tcagggcgcg tcagcgggtg ttggcgggtg tcggggctgg 240
cttaactatg agtcccgcgc agtcgcccac aaccgcccac agccccgacc gaattgatac 300
cggcatcaga gcagattgta ctgagagtgc accatatatg cggtgtgaaa gccgtagtct 360
cgtctaacat gactctcacg tggtatatac gccacacttt taccgcacag atgcgtaagg 420
agaaaatacc gcatcaggcg ccattcgcca atggcgtgtc tacgcattcc tcttttatgg 480
cgtagtccgc ggtaagcggt ttcaggctgc gcaactgttg ggaagggcga tcggtgcggg 540
cctcttcgct aagtccgacg cgttgacaac ccttcccgct agccacgccc ggagaagcga 600
attacgccag ctggcgaaag ggggatgtgc tgcaaggcga ttaagttggg taatgcggtc 660
gaccgctttc cccctacacg acgttccgct aattcaaccc taacgccagg gttttcccag 720
tcacgacgtt gtaaaacgac ggccagtgcc attgcggtcc caaaagggtc agtgctgcaa 780
cattttgctg ccggtcacgg aagcttgcat gcaaggagat ggcgcccaac agtcccccgg 840
ccacggggcc ttcgaacgta cgttcctcta ccgcgggttg tcagggggcc ggtgccccgg 900
tgccaccata cccacgccga aacaagcgct catgagcccg aagtggcgag acggtggtat 960
gggtgcggct ttgttcgcga gtactcgggc ttcaccgctc cccgatcttc cccatcggtg 1020

atgtcggcga tataggcgcc agcaaccgca gggctagaag gggtagccac tacagccgct 1080
atatccgcgg tcgttggcgt cctgtggcgc cggtgatgcc ggccacgatg cgtccggcgt 1140
agaggatcga ggacaccgcg gccactacgg ccggtgctac gcaggccgca tctcctagct 1200
gatctcgatc ccgcgaaatt aatacgactc actataggga gaccacaacg ctagagctag 1260
ggcgctttaa ttatgctgag tgatatccct ctggtgttgc gtttccctct agaaataatt 1320
ttgtttaact ttaagaagga gatataccat caaaggagaa tctttattaa aacaaattga 1380
aattcttcct ctatatggta gagtggttta aacgatattt tcgaggctca gaaaatcgaa 1440
tggcacgaaa ctcaccaaat ttgctataaa agctccgagt cttttagctt accgtgcttt 1500
tcgaaggccg cggccgctta attaaacata tgaccatggt tatcgaaggc agcttccggc 1560
gccggcgaat taatttgtat actggtacca atagcttccg cgcgccccac cacgcctcat 1620
ctgtgacagc cgagtcctgg agaggtacct gcgcggggtg gtgcggagta gacactgtcg 1680
gctcaggacc tctccatgga cttggaggcc aaggaggccg agaatatcac gacgggctgt 1740
gctgaacact gaacctccgg ttcctccggc tcttatagtg ctgcccgaca cgacttgtga 1800
gcagcttgaa tgagaatatc actgtcccag acaccaaagt taatttctat cgtcgaactt 1860
actcttatag tgacagggtc tgtggtttca attaaagata gcctggaaga ggatggaggt 1920
cgggcagcag gccgtagaag tctggcaggg cggaccttct cctacctcca gcccgtcgtc 1980
cggcatcttc agaccgtccc cctggccctg ctgtcggaag ctgtcctgcg gggccaggcc 2040
ctgttggtca ggaccgggac gacagccttc gacaggacgc cccggtccgg gacaaccagt 2100
actcttccca gccgtgggag cccctgcagc tgcatgtgga taaagccgtc tgagaagggt 2160
cggcaccctc ggggacgtcg acgtacacct atttcggcag agtggccttc gcagcctcac 2220
cactctgctt cgggctctgg gagcccagaa tcaccggaag cgtcggagtg gtgagacgaa 2280
gcccgagacc ctcgggtctt ggaagccatc tcccctccag atgcggcctc agctgctcca 2340
ctccgaacaa ccttcggtag aggggaggtc tacgccggag tcgacgaggt gaggcttgtt 2400
tcactgctga cactttccgc aaactcttcc gagtctactc caatttcctc agtgacgact 2460
gtgaaaggcg tttgagaagg ctcagatgag gttaaaggag cggggaaagc tgaagctgta 2520
cacaggggag gcctgcagga caggggacag gcccctttcg acttcgacat gtgtcccctc 2580
cggacgtcct gtcccctgtc atgataaccc gggatccggt aagatccggc tgctaacaaa 2640
gcccgaaagg tactattggg ccctaggcca ttctaggccg acgattgttt cgggctttcc 2700
aagctgagtt ggctgctgcc accgctgagc aataactagc ataacccctt ttcgactcaa 2760
ccgacgacgg tggcgactcg ttattgatcg tattggggaa ggggcctcta aacgggtctt 2820
gaggggtttt ttgctgaaag gaggaactat ccccggagat ttgcccagaa ctccccaaaa 2880
aacgactttc ctccttgata atccggatat ccacaggacg ggtgtggtcg ccatgatcgc 2940
gtagtcgata taggcctata ggtgtcctgc ccacaccagc ggtactagcg catcagctat 3000
gtggctccaa gtagcgaagc gagcaggact gggcggcggc caaagcggtc caccgaggtt 3060
catcgcttcg ctcgtcctga cccgccgccg gtttcgccag ggacagtgct ccgagaacgg 3120
gtgcgcatag aaattgcatc aacgcatata cctgtcacga ggctcttgcc cacgcgtatc 3180
tttaacgtag ttgcgtatat gcgctagcag cacgccatag tgactggcga tgctgtcgga 3240
atggacgata cgcgatcgtc gtgcggtatc actgaccgct acgacagcct tacctgctat 3300
tcccgcaaga ggcccggcag taccggcata accaagccta tgcctacagc agggcgttct 3360
ccgggccgtc atggccgtat tggttcggat acggatgtcg atccagggtg acggtgccga 3420
ggatgacgat gagcgcattg ttagatttca taggtcccac tgccacggct cctactgcta 3480
ctcgcgtaac aatctaaagt tacacggtgc ctgactgcgt tagcaattta actgtgataa 3540
actaccgcat atgtgccacg gactgacgca atcgttaaat tgacactatt tgatggcgta 3600
taaagcttat cgatgataag ctgtcaaaca tgagaattcg taatcatgtc atttcgaata 3660
gctactattc gacagtttgt actcttaagc attagtacag atagctgttt cctgtgtgaa 3720
attgttatcc gctcacaatt ccacacaaca tatcgacaaa ggacacactt taacaatagg 3780
cgagtgttaa ggtgtgttgt tacgagccgg aagcataaag tgtaaagcct ggggtgccta 3840
atgagtgagc atgctcggcc ttcgtatttc acatttcgga ccccacggat tactcactcg 3900
taactcacat taattgcgtt gcgctcactg cccgctttcc agtcgggaaa attgagtgta 3960
attaacgcaa cgcgagtgac gggcgaaagg tcagcccttt cctgtcgtgc cagctgcatt 4020
aatgaatcgg ccaacgcgcg gggagaggcg ggacagcacg gtcgacgtaa ttacttagcc 4080
ggttgcgcgc ccctctccgc gtttgcgtat tgggcgctct tccgcttcct cgctcactga 4140
ctcgctgcgc caaacgcata acccgcgaga aggcgaagga gcgagtgact gagcgacgcg 4200
tcggtcgttc ggctgcggcg agcggtatca gctcactcaa aggcggtaat agccagcaag 4260
ccgacgccgc tcgccatagt cgagtgagtt tccgccatta acggttatcc acagaatcag 4320
gggataacgc aggaaagaac atgtgagcaa tgccaatagg tgtcttagtc ccctattgcg 4380

tcctttcttg tacactcgtt aaggccagca aaaggccagg aaccgtaaaa aggccgcgtt 4440
gctggcgttt ttccggtcgt tttccggtcc ttggcatttt tccggcgcaa cgaccgcaaa 4500
ttccataggc tccgcccccc tgacgagcat cacaaaaatc gacgctcaag aaggtatccg 4560
aggcgggggg actgctcgta gtgtttttag ctgcgagttc tcagaggtgg cgaaacccga 4620
caggactata aagataccag gcgtttcccc agtctccacc gctttgggct gtcctgatat 4680
ttctatggtc cgcaaagggg ctggaagctc cctcgtgcgc tctcctgttc cgaccctgcc 4740
gcttaccgga gaccttcgag ggagcacgcg agaggacaag gctgggacgg cgaatggcct 4800
tacctgtccg cctttctccc ttcgggaagc gtggcgcttt ctcatagctc atggacaggc 4860
ggaaagaggg aagcccttcg caccgcgaaa gagtatcgag acgctgtagg tatctcagtt 4920
cggtgtaggt cgttcgctcc aagctgggct tgcgacatcc atagagtcaa gccacatcca 4980
gcaagcgagg ttcgacccga gtgtgcacga accccccgtt cagcccgacc gctgcgcctt 5040
atccggtaac cacacgtgct tggggggcaa gtcgggctgg cgacgcggaa taggccattg 5100
tatcgtcttg agtccaaccc ggtaagacac gacttatcgc cactggcagc atagcagaac 5160
tcaggttggg ccattctgtg ctgaatagcg gtgaccgtcg agccactggt aacaggatta 5220
gcagagcgag gtatgtaggc ggtgctacag tcggtgacca ttgtcctaat cgtctcgctc 5280
catacatccg ccacgatgtc agttcttgaa gtggtggcct aactacggct acactagaag 5340
gacagtattt tcaagaactt caccaccgga ttgatgccga tgtgatcttc ctgtcataaa 5400
ggtatctgcg ctctgctgaa gccagttacc ttcggaaaaa gagttggtag ccatagacgc 5460
gagacgactt cggtcaatgg aagccttttt ctcaaccatc ctcttgatcc ggcaaacaaa 5520
ccaccgctgg tagcggtggt ttttttgttt gagaactagg ccgtttgttt ggtggcgacc 5580
atcgccacca aaaaaacaaa gcaagcagca gattacgcgc agaaaaaaag gatctcaaga 5640
agatcctttg cgttcgtcgt ctaatgcgcg tctttttttc ctagagttct tctaggaaac 5700
atcttttcta cggggtctga cgctcagtgg aacgaaaact cacgttaagg tagaaaagat 5760
gccccagact gcgagtcacc ttgcttttga gtgcaattcc gattttggtc atgagattat 5820
caaaaaggat cttcacctag atccttttaa ctaaaaccag tactctaata gtttttccta 5880
gaagtggatc taggaaaatt attaaaaatg aagttttaaa tcaatctaaa gtatatatga 5940
gtaaacttgg taatttttac ttcaaaattt agttagattt catatatact catttgaacc 6000
tctgacagtt accaatgctt aatcagtgag gcacctatct cagcgatctg agactgtcaa 6060
tggttacgaa ttagtcactc cgtggataga gtcgctagac tctatttcgt tcatccatag 6120
ttgcctgact ccccgtcgtg tagataacta agataaagca agtaggtatc aacggactga 6180
ggggcagcac atctattgat cgatacggga gggcttacca tctggcccca gtgctgcaat 6240
gataccgcga gctatgccct cccgaatggt agaccggggt cacgacgtta ctatggcgct 6300
gacccacgct caccggctcc agatttatca gcaataaacc agccagccgg ctgggtgcga 6360
gtggccgagg tctaaatagt cgttatttgg tcggtcggcc aagggccgag cgcagaagtg 6420
gtcctgcaac tttatccgcc tccatccagt ttcccggctc gcgtcttcac caggacgttg 6480
aaataggcgg aggtaggtca ctattaattg ttgccgggaa gctagagtaa gtagttcgcc 6540
agttaatagt gataattaac aacggccctt cgatctcatt catcaagcgg tcaattatca 6600
ttgcgcaacg ttgttgccat tgctacaggc atcgtggtgt cacgctcgtc aacgcgttgc 6660
aacaacggta acgatgtccg tagcaccaca gtgcgagcag gtttggtatg gcttcattca 6720
gctccggttc ccaacgatca aggcgagtta caaaccatac cgaagtaagt cgaggccaag 6780
ggttgctagt tccgctcaat catgatcccc catgttgtgc aaaaaagcgg ttagctcctt 6840
cggtcctccg gtactagggg gtacaacacg ttttttcgcc aatcgaggaa gccaggaggc 6900
atcgttgtca gaagtaagtt ggccgcagtg ttatcactca tggttatggc tagcaacagt 6960
cttcattcaa ccggcgtcac aatagtgagt accaataccg agcactgcat aattctctta 7020
ctgtcatgcc atccgtaaga tgcttttctg tcgtgacgta ttaagagaat gacagtacgg 7080
taggcattct acgaaaagac tgactggtga gtactcaacc aagtcattct gagaatagtg 7140
tatgcggcga actgaccact catgagttgg ttcagtaaga ctcttatcac atacgccgct 7200
ccgagttgct cttgcccggc gtcaatacgg gataataccg cgccacatag ggctcaacga 7260
gaacgggccg cagttatgcc ctattatggc gcggtgtatc cagaacttta aaagtgctca 7320
tcattggaaa acgttcttcg gggcgaaaac gtcttgaaat tttcacgagt agtaaccttt 7380
tgcaagaagc cccgcttttg tctcaaggat cttaccgctg ttgagatcca gttcgatgta 7440
acccactcgt agagttccta gaatggcgac aactctaggt caagctacat tgggtgagca 7500
gcacccaact gatcttcagc atcttttact ttcaccagcg tttctgggtg cgtgggttga 7560
ctagaagtcg tagaaaatga aagtggtcgc aaagacccac agcaaaaaca ggaaggcaaa 7620
atgccgcaaa aaagggaata agggcgacac tcgtttttgt ccttccgttt tacggcgttt 7680
tttcccttat tcccgctgtg ggaaatgttg aatactcata ctcttccttt ttcaatatta 7740

ttgaagcatt cctttacaac ttatgagtat gagaaggaaa aagttataat aacttcgtaa 7800
tatcagggtt attgtctcat gagcggatac atatttgaat gtatttagaa atagtcccaa 7860
taacagagta ctcgcctatg tataaactta cataaatctt aaataaacaa ataggggttc 7920
cgcgcacatt tccccgaaaa gtgccacctg tttatttgtt tatccccaag gcgcgtgtaa 7980
aggggctttt cacggtggac acgtctaaga aaccattatt atcatgacat taacctataa 8040
aaataggcgt tgcagattct ttggtaataa tagtactgta attggatatt tttatccgca 8100
atcacgaggc cctttcgtct agtgctccgg gaaagcag 8138